(19) Europäisches Patentamt
European Patent Office
Office européen des brevets

(11) Publication number: **0 170 697 B1**

(12) **EUROPEAN PATENT SPECIFICATION**

(45) Date of publication of patent specification: **23.10.91**

(51) Int. Cl.[5]: **A61K 39/44**, A61K 47/48, C07K 17/06, C12N 15/11, C12N 15/29

(21) Application number: **85901197.5**

(22) Date of filing: **07.02.85**

(86) International application number: **PCT/US85/00197**

(87) International publication number: **WO 85/03508 (15.08.85 85/18)**

Divisional application 89201162.8 filed on 07/02/85.

(54) **TOXIN CONJUGATES.**

(30) Priority: **08.02.84 US 578115**
**08.02.84 US 587121**
**09.02.84 US 578122**
**07.09.84 US 648759**
**20.09.84 US 653515**

(43) Date of publication of application: **12.02.86 Bulletin 86/07**

(45) Publication of the grant of the patent: **23.10.91 Bulletin 91/43**

(84) Designated Contracting States: **AT BE CH DE FR GB LI LU NL SE**

(56) References cited:
**EP-A- 23 401**
**EP-A- 23 779**
**EP-A- 44 167**
**EP-A- 55 115**
**EP-A- 55 575**
**EP-A- 319 99**
**WO-A-83/03971**
**FR-A- 2 437 213**
**US-A- 4 359 457**

(73) Proprietor: **CETUS CORPORATION**
**1400 Fifty-Third Street**
**Emeryville California 94608(US)**

(72) Inventor: **GREENFIELD, Lawrence, I.**
**555 Pierce Street, No. 435**
**Albany, CA 94706(US)**
Inventor: **NITECKI, Danute, E.**
**2296 Virginia Street**
**Berkeley, CA 94709(US)**
Inventor: **KAPLAN, Donald, A.**
**3301 Noeske Drive**
**Midland, MI 48640(US)**
Inventor: **GELFAND, David, H.**
**6208 Chelton Drive**
**Oakland, CA 94611(US)**
Inventor: **PIATAK, Michael**
**1120 Alfred Avenue**
**Walnut Creek, CA 94596(US)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services

Chemical abstracts, vol, 98, n 9, 28 February 1983 (Colombus, Ohio, US) see page 54, abstract n 65458y & US 350223 (United States Dept. of Health and Human Services), 3 December 1982

Nature, vol. 255, 5 June 1975 ; G.F. Rowland et al "Suppression of tumor growth in mice by a drugantibody conjugate using a novel approach to linkage", pages 487-488, see the entire article.

Proc. Natl, Cad, Sci. USA, vol. 79, January 1982, Medical Sciences ; A. Trouet et al "A covalent linkage between daunorubicin and proteins that is table in serum and reversible by lysosomal hydrolases, as required for a lysosomotropic drug-carrier conjugate : in vitro and in vivo studies", pages 626-629, see pages 626 and 627 "Material and methods"

Proc. Natl. Acad. Sci. USA, vol. 80, November 1983, Biochemistry, L. Greenfield et al "Nucleotide sequence of the structural gene for diphtheria toxin carried by corynebacteriophage B", pages 6853-6857

See also references of WO8503508

Inventor: HORN, Glenn
3 Admiral Drive
Emeryville, CA 94608(US)
Inventor: LAWYER, Francis Cook
6641 Saroni Drive
Oakland, CA 94611(US)

(74) Representative: Bizley, Richard Edward et al
HEPWORTH LAWRENCE BRYER & BIZLEY
2nd Floor Gate House South West Gate
Harlow, Essex CM20 1JN(GB)

## Description

### Technical Field

This invention relates to immunotoxin conjugates, fused polypeptides for making such conjugates, and the use of such conjugates. More specifically, the invention relates to spacers for such conjugates and includes methods for preparing such spacers.

### Background Art

Bacterial and plant toxins, such as diphtheria toxin (DT), Pseudomonas aeruginosa toxin A, abrin, ricin, mistletoe, modeccin, and Shigella toxin, are potent cytocides due to their ability to disrupt a critical cellular function. For instance, DT and ricin inhibit cellular protein synthesis by inactivation of elongation factor-2 and inactivation of ribosomal 60s subunits, respectively. Bacterial Toxins and Cell Membranes, Eds. Jelajaszewicz, J. and Wadstrom, T. (1978) Academic Press, p. 291. These toxins are extremely potent because they are enzymes and act catalytically rather than stoichiometrically. The molecules of these toxins are composed of an enzymatically active polypeptide chain or fragment, commonly called an "A" chain or fragment, linked to one or more polypeptide chains or fragments, commonly called "B" chains or fragments, that bind the molecule to the cell surface and enable the A chain to reach its site of action, e.g., the cytosol, and carry out its disruptive function. The act of gaining access to the cytosol is called variously "internalization", "intoxication", or "translocation". It is believed that the A chain must be timely liberated from the B chain--frequently by reduction of a disulfide bond--in order to make the A chain functional. These natural toxins are generally not selective for a given cell or tissue type because their B chains recognize and bind to receptors that are present on a variety of cells.

### Toxin Delivery Design Strategies

Derivatives of these bacterial and plant toxins have been prepared as therapeutic agents, primarily as antineoplastic agents, that are made specific for tumor cells or other target cells by replacing the native B chain(s) of the toxin molecule with a surrogate B chain that is specific for the tumor cell or adding a B chain having such specificity to the toxin molecule. Pharmacology of Bacterial Toxins, Eds. Drews, J. and Dorner, F. Pergamon Press. Synthetic cytotoxins containing the active fragment of a bacterial or plant toxin or a cytotoxic drug are called variously "chimeric toxins", "toxin conjugates", "cytotoxic conjugates", "hybrid toxins" or, when the surrogate B moiety is an antibody, "immunotoxins". Antibodies (polyclonal, monoclonal, and antigen binding fragments), hormones, lectins and various other compounds that are recognized by receptors on tumor cell surfaces have been used as surrogate B moieties. See European patent application publication no 0044167, and US Pats Nos 4,340,535, 4,350,626, 4,357,273, 4,359,457 4,363,758, 4,368,149, and 4,379,145.

Surrogate B moieties have been chemically linked to toxin A chains by a variety of coupling agents. Heterobifunctional agents that include a disulfide group have been used extensively. The most popular of these agents is N-succinimidyl-3-(2-pyridyldithio)propionate (SPDP). Immun Rev, 62:185-216 (1982) reports using a longer disulfide-containing coupling agent, 7-aza-8-oxo-10-(2-pyridyldithio)decanoic acid, to investigate whether greater separation between the A chain and antibody would increase activity. Increased activity was observed in an acellular system but not on intact cells, and the report concluded that the longer disulfide containing linker was not advantageous. Replacement of the disulfide bridge by a stable thioether bridge using a derivative of 6-maleimidocaproic acid as a bifunctional coupling agent for an A-antibody conjugate caused a 99% loss of activity relative to the disulfide conjugate in an intact cellular system.

The toxicity of diphtheria toxin for human lymphoblastoid cells was increased by covalent linkage to anti-lymphoblastoid (anti-CLA 4 and anti-Daudi) globulin. (Ross, W.C.J., et al, Eur J Biodiem (1980) 104:381). Thyrotropin releasing hormone derivatives have also been conjugated to DT fragments such as CRM 45 to study the translocation function (Bacha, D., et al, J Biol Chem (1983) 238:1565). DT-A conjugated using SPDP to a monoclonal antibody against guinea pig hepatocarcinoma cells showed specific cytotoxicity in vitro and in vivo (Bernhard, M.I. et al, Cancer Res (1983) 43:4420).

Dextran, polyglutamic acid, and oligopeptides containing up to four amino acid residues have been used as spacer arm bridges between cytotoxic drugs and antibodies. Nature (1975) 255:487-488, FEBS letters (1980) 119:181-186, PNAS (1982) 79:626-629, and Immun Rev, 62:1-27 (1982). These conjugates were reported to have higher toxicity in vitro than drug coupled directly to antibody.

Moolten, F.L., et al, Immun Rev, (1982) 62:47-72 conclude that A chain-antibody conjugates may be

more specific than native toxins, but lack much of the potency of the native toxin. They speculate that the loss of efficacy is because the internalization function is present in native B chains and surrogate B chains are inefficient substitutes as regards internalization. Use of toxins that lack a binding function but are otherwise intact, such as the (cross reacting mutant) CRM45 of DT or toxins whose binding function is chemically or enzymatically abrogated, are suggested, but no evidence of increased efficacy is given.

In summation, the efficacy of prior toxin-antibody conjugates has been highly variable due, inter alia, to variations in immunogenicity, target cell specificity, nonspecific toxicity, serum stability, effective concentration at the target cell, binding efficiency, and internalization efficiency. The improved immunotoxins of this invention provide (1) means for improving the efficacy of toxin-antibody conjugates and (2) novel toxin conjugates that include those means, by providing an A - surrogate B geometry which permits more facile translocation of the A portion into the target cell, and a more stable mode by which antibody can be linked to the A portion. This latter property prevents premature decomposition prior to translocation of the A portion into the target cell.

The present invention provides novel toxin conjugates which are peculiarly effective in recognizing target cells and in effecting their demise. It also provides other components of these conjugates. The conjugates comprise a cytotoxic component which is an enzymatically active portion of the molecule, capable of killing cells in which it is internalized, a specific binding moiety, typically an antibody or fragment of an antibody, which is capable of recognizing a specific antigenic determinant or target cell, and a spacer which provides the proper geometry between the cytotoxic component and the binding fragment. These conjugates represent an improved delivery system for naturally occuring or modified cytotoxins ("A chains") which in their natural environment are bound to a relatively non-specific binding component "B chain" (which is generally not an antibody). The naturally occuring toxins also contain, within the A-B chain fusion, sequences which are capable of cleavage intracellularly, ie which effect cleavage once the cytotoxic component has migrated to within the target cell, but are stable prior to this entry, and a translocation region which permits the desired cytotoxic A chain to enter the target cell. This intracellular cleavage exposes and labilizes the link (typically disulfide) between the A and B chain.

In the conjugates of the present invention, the non-binding portion of the molecule is constructed so as to retain the foregoing desired cytotoxic, intracellularly cleavable/extracellularly stable, and translocation properties of the natural molecule in a geometry suitable for connecting to a "binding fragment" and permitting activity. Thus, typically, the conjugates of the invention include: antibody or fragment of an antibody which is covalently linked, preferably through a bifunctional linker to a non-binding entity. The non-binding entity is an amino acid sequence which contains, to serve as the cytotoxic component, an enzymatically active site, an intracellularly cleavable/extracellularly stable site and translocation sequence and, as an extension of this amino acid sequence, a further sequence which serves as a spacer between the cytotoxic component and the binding fragment to be linked. The non-binding portion of the toxin conjugates of the invention may be supplied using recombinant techniques.

The spacer confers the additional advantage of enhanced solubility in some instances where intermediates for desired immunotoxin may be sufficiently insoluble to interfere with their purification. By supplying a relatively more soluble portion, or by altering the conformation of the remainder of the molecule, the spacer thus permits more options in the selection of components and conjugation methods.

The invention, therefore, relates to these conjugates and to the novel components used for their construction including especially those formed by recombinant means. Both the spacer segment and the non-binding portion of the conjugate formed by fusion of the spacer with a cytotoxic component, ie, an extended spacer containing the cytotoxic component are aspects of the invention.

Thus, in one aspect, the invention relates to novel polypeptides (the spacer) consisting essentially of at least one rigid amino acid sequence bracketed by two flexible amino acid sequences--ie, components of the formula $(\text{flex-rigid})_n\text{flex}$, where "flex" represents the flexible sequence and "rigid" the rigid sequence. In a preferred embodiment "flex" is about 4 to 8 amino acids each individually selected from the group consisting of threonine, serine and glycine and "rigid" is 4 to 8 proline residues. Such novel polypeptides are useful as spacers for separating the cytotoxic component and the target cell binding component of a toxin conjugate.

In the alternative, the spacer may be described in functional terms and comprises an amino acid sequence that:

(a) is substantially stable in serum;

(b) is substantially nonhydrophobic so as not to affect adversely the water solubility of the toxin conjugate;

(c) is at least about 15 A long;

(d) has a substantially extended structure; and

(e) is sufficiently flexible to permit three dimensional movement of the cytotoxic component and the target cell binding component.

The spacer may also be a solubilization conferring sequence of amino acids, as set forth hereinbelow.

The foregoing spacer descriptions are not mutually exclusive, but are alternative characterizations of successful peptide sequences. The spacer may further contain a reactive amino acid residue proximate one of its termini so as to provide a conjugation site for the additional binding fragment.

Other aspects of the invention are fused polypeptides for use in making toxin conjugates comprising:

(a) a cytotoxic portion; and

(b) one of the above-described polypeptide spacers, as well as recombinant peptides which are useful cytotoxic moieties for the conjugates.

The invention also concerns the DNA sequences encoding the spacer, toxin fragments, or fused polypeptide (cytotoxic/spacer); expression vectors containing these DNA sequences, and cells transformed with these vectors.

Still another aspect of the invention is a toxin conjugate which comprises:

(a) a cytotoxic portion;

(b) a polypeptide spacer bound to the cytotoxic component; and

(c) a target cell binding moiety conjugated to the cytotoxic component via the polypeptide spacer.

(The "cytotoxic portion" includes a site for intracellular cleavage within a serum stable domain and an internalization facilitating domain.)

In summary, the invention is designed to provide a toxin conjugate which has the appropriate geometry for translocating the cytotoxic fragment into the target cell, the capacity to retain its binding fragment prior to such translocation, and/or the ability to solubilize the cytotoxic portion. In one aspect of the invention, the spacer is designed so as to permit the cytotoxic portion of the molecule ready access to the cell membrane. As the size of a typical antibody (binding) fragment is very much greater than that of most cytotoxic fragments, there is considerable steric hinderance of the access to the cell membrane by the cytotoxic portion imposed by the sheer bulk of the antibody or antibody fragments. Accordingly, the conjugate toxins of the invention have a geometry schematically represented in (a) rather than that given without the spacer (b).

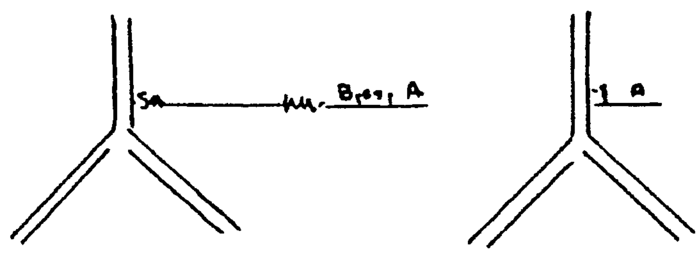

(a) (b)

In order to effect this translocation, the spacer needs to be sufficiently flexible to allow the A portion to reach the cell membrane, and sufficiently extended to permit it to have sufficient reach.

The performance of the conjugated toxin can also be improved by securing the binding portion tightly to the remainder of the molecule with respect to a serum environment. This is done in one preferred embodiment of the invention, by utilizing a linker between the antibody and spacer which employs bonds not readily cleaved by reducing agents or by hydrolysis under extracellular conditions. The cleavage of the A-chain analog (ie the enzymatically active site) from the other end of the spacer arm can be achieved by permitting the linkage at the A end of the spacer to be more readily cleavable. This can best be done by retaining a portion of the original B chain of the native toxin in linking the A portion to the spacer. In this manner, the normal extracellular-resistant/intracellular-cleavable configuration of the A-B chain pair is retained but with the loss of the binding capacity of the original B portion. Thus, the specific binding capability conferred by the antibody on the conjugate is not lost prior to intracellular incorporation of the cytotoxic fragment. Such flexibility in the nature of the non-binding moieties available is provided through the recombinant methods of the invention.

Since the binding portion confers specific recognition of certain target cells, the conjugated toxins are useful in killing specified undesirable cells within a subject. Thus, in two other aspects, the invention relates to pharmaceutical compositions containing effective amounts of these toxins and to methods of treatment

employing them.

Brief Description of the Drawings

Figure 1 shows the nucleotide sequence of the DT gene along with the corresponding deduced amino acid sequence.

Figure 2 shows the construction of an Msp-Spacer arm clone, pMspSA2.

Figure 3 shows the construction of two Msp-Spacer fragment expression vectors wherein the coding sequence is under the control of the $P_L$ promoter: $pP_L$MspSA and $pP_L$OPMspSA2.

Figure 4 shows the construction of pTrpSmlMbo.

A. Definitions

As used herein the terms "fragment", "domain", and "region" and "portion" are interchangeable and refer to functionally but not necessarily physically distinct portions of the conjugated toxin molecule.

The term "specificity" as used to describe the target cell binding portion of the conjugated toxin means that the moiety has the ability to distinguish a target cell from other cells, typically due to the presence of a cell surface receptor that is unique to the target cells.

The term "selective" means that the cytotoxin has the ability to kill target cells preferentially, typically due to the specificity of the binding moiety or a differential in the respective quantities of receptors on target cells and other cells.

The term "target cells" means those cells which the cytotoxin is intended to kill. Although target cells will usually be tumor cells, they may be nontumorous cells whose selective destruction is desired for therapeutic or diagnostic purposes (for instance in certain assays of peripheral blood cells it is desired to selectively kill one or the other of B cells or T cells). The target cells may be present in living organisms or they may be preserved or maintained in vitro. The cells may be individual or associated to form an organ.

As used herein the term "polypeptide" or "protein" refers to an amino acid polymer. It is understood that such polymers exist in a variety of ionization states dependent on ambient pH, and that they may, further, be associated with accessory moieties--eg, glycosylated, phosphorylated or conjugated to lipids. The peptides or proteins of the invention include all such forms, including unassociated forms.

The term "intracellular" is intended to include intracytoplasmic sites and sites within vesicular compartments such as lysosomes.

"Target cell binding portion" refers to that fragment of the toxin conjugates of the invention which binds specifically to the target cells. In this invention the "binding" portion is an antibody or fragment thereof. The "non-binding" portion or fragment of the toxin conjugate comprises the remainder of the molecule. It thus includes the cytotoxic portion and the spacer.

The term "cytotoxic portion" includes the "enzymatically active polypeptide fragment" ie an A-fragment analogous sequence, the intracellularly cleavable/extracellularly stable domain and the translocation domain.

As used herein with respect to the construction of the spacer domain, "reactive amino acid residue" refers to an amino acid (or residue) which provides a site for linking or conjugation, as further described below.

"Solubilizing conferring" sequence of amino acids in the context of the invention refers to a form of spacer sequence which continues at the (preferably C) terminus of the cytotoxic portion and which results in the fused protein being soluble in aqueous media, even when the cytotoxic portion is itself insoluble or when the cytotoxic portion would otherwise be rendered insoluble by the addition of a cysteine residue.

As used herein "DT-A fragment" (diphtheria toxin-A fragment) refers to an approximately 193 residue amino acid sequence which is capable of ADP-ribosylation of EF-2, and which is substantially similar to the sequence in Figure 1 between amino acid 1 and amino acid 192 or 193.

"DT-B fragment" is an approximately 343 residue amino acid sequence which is substantially similar to the sequence approximately between amino acids 193 and 535 in Figure 1.

"DT-A-B' fragment" is an approximately 384 residue amino acid sequence which is capable of ADP-ribosylation of EF-2, which contains both a translocation sequence and an intracellular cleavage site within an extracellularly stable domain, and which is substantially similar to the sequence shown in Figure 1 between amino acid 1 and amino acid 384.

"Msp fragment" (fragment 2) refers to an approximately 1454 bp segment which contains all of the A portion and part of the B portion--i.e., a DT-A-B' fragment along with at least part of native promoter, ribosome binding site and secretory leader sequence as shown in Figure 1. "Msp terminator" refers to a nucleotide sequence (synthetically derived herein) which encodes one additional amino acid past the Msp

cleavage point plus a stop codon. The resultant (Msp + Msp terminator) encodes a "DT-A-B' fragment."

"Mbo fragment" (fragment 1) refers to an approximately 831 bp resultant of an Mbo digest of the DT gene. It contains the coding sequence of amino acids 1-193 (the A chain) and leader and at least partial native control sequences (see Fig. 1). "Mbo terminator" refers to a nucleotide sequence (synthetically derived herein) which encodes an additional six amino acids past the Mbo cleavage plus a stop codon. The resultant (Mbo + Mbo terminator) encodes a "DT-A fragment."

"Native" promoter, ribosome binding site, or control sequences refer to those which are normally found in association with, and operably linked to, the DT coding sequence. "Leader" sequence refers to that portion of the DNA which encodes the native pre-sequence, responsible for secretion of the mature protein.

As used herein, "ricin A" refers to a protein whose amino acid sequence is substantially similar to that of the ricin A peptide which is extractable from castor bean seeds. The ricin A of castor beans is approximately 265 amino acids in length and has a molecular weight of approximately 32,000 daltons. However, it is known that the precise sequence varies depending on the variety of bean, and, indeed that at least two slightly different forms of ricin A may be present in a single variety.

"Ricin B" refers to a protein whose amino acid sequence is substantially similar to that of the ricin B peptide which is extractable from castor bean seeds. The ricin B of castor beans is approximately 260 amino acids in length and has a molecular weight of approximately 34,700 daltons; as with ricin A, it is known that the precise sequence varies depending on the variety of bean.

"Substantially similar" means that the protein in question must be approximately the same length (arbitrarily within around 10%) but, more importantly, must retain the capacity of reference chain to carry out its native biological activity. It is well known that some small alterations in protein sequence may be possible without disturbing the functional abilities of the protein molecule, although other modifications are totally destructive. It is not currently possible to predict with any assurance into which category a particular alteration will fall. The definition herein permits any modifications which are in the first category. Such alterations could result from chance mutations in the gene sequence or from deliberate alterations thereof.

Further, as is well known, protein sequences may be modified by post-translational processing such as association with other molecules, for example, glycosides, lipids, or such inorganic ions as phosphate. The ionization status will also vary depending on the pH of the medium or the pH at which crystallisation or precipitation of the isolated form occurs. Further, the presence of air may cause oxidation of labile groups, such as -SH. Included within the definition of the peptides defined herein are all such modifications of a particular primary structure--ie, eg, both glycosylated and non-glycosylated forms, neutral forms, acidic and basic salts, lipid or other associated peptide forms, side chain alterations due to oxidation or derivatization, and any other such modifications of an amino acid sequence which would be encoded by the same genetic codon sequence.

"Ricin" refers to peptides which contain both A and B chains, defined respectively as set forth herein. The peptide may mimic the native peptide in that the A and B proteins are linked only by a disulfide, or may contain the intermediate dodecamer or other short peptide sequence between the A and B chains.

"Operably linked" when used in describing DNA sequences refers to juxtaposition in such a way that the functionality of the sequences is preserved. Thus, for example a coding sequence "operably linked" to control sequences is positioned so that the these sequences are capable of effecting the expression of the coding sequence.

"Control" sequence refers to those DNA sequences which control initiation and termination of transcription and translation. In procaryotic systems, for example, control sequences comprise promoter or promoter/operator and nucleotides encoding a ribosome binding site; in eucaryotes, promoters, terminators and enhancers appear to be involved.

B. Components of Conjugate Toxins

Toxin conjugates have classically been conceptualized as combinations of an A fragment and a surrogate B moiety, attached through linking group that binds these fragments via a labile bridge such as a disulfide bridge. The rationale behind this conceptualization was that the function of the B moiety was primarily to bind the conjugate to the cell surface via interaction with a cell surface receptor and that the disulfide bridge provided means for joining the A fragment and B moiety that could be broken in vivo to liberate the A fragment. The present invention is based on an expanded conceptualization of the mode of operation of cytotoxic conjugates and takes into account factors including:

- the spatial relationship between the cytotoxic moiety and the binding moiety,
- the role of the non-A portion of the conjugate in internalization, and
- the extracellular lability of the bond between the cytotoxic moiety and the binding moiety.

In the most preferred embodiment of the present invention with respect to toxin conjugates each of these factors is taken into account in synthesizing a novel toxin conjugate having five functional elements (which may overlap in the structure of the conjugate):

(1) an enzymatically active domain capable of the toxic activity of the A-fragment;

(2) an intracellular cleavage site within a extracellularly stable (or serum stable) domain that provides a site for liberating the enzymatically active domain from the remainder of the toxin conjugate after the toxin conjugate has been internalized;

(3) a translocation or internalization facilitating domain that acts as an adhesive to anchor the toxin conjugate molecule to the target cell membrane and/or to facilitate internalization;

(4) the novel polypeptide spacer described above; and

(5) a target cell binding moiety that recognizes and binds to a receptor on the target cell surface which by virtue of the receptor's quantity or nature causes the toxin conjugate to localize selectively on target cells relative to other cells.

In the most preferred embodiment the binding moiety is bound to the non-binding portion of the molecule (ie the remaining elements 1-4) by a chemical bond that is substantially resistant to cleavage in vivo (either extracellularly or intracellularly) thereby preserving the specificity of the molecule once it is administered, while the intracellular cleavage site within a serum stable domain is provided at the enzymatically active or "A" end of the spacer. In order to effect this linkage using one preferred embodiment the cytotoxic portion of the toxin needs to be provided with a free cysteine residue, capable of forming a thio-ether linkage through a suitable bifunctional linker. The presence of this cysteine may interfere with the solubility of the toxic portion, and the spacer then serves the additional function of providing solubilization. To accomplish this purpose, the detailed "spatial" requirements relating to rigidity and flexibility of the sequence are not required; all that is needed are the hydrophilic or neutral solubility properties of the chain.

B.1. The Enzymatically Active Domain

The enzymatically active fragment of the conjugate may be the A chain of a bacterial or plant toxin or be a natural protein that has enzymatic activity similar to the A chain of a bacterial or plant toxin. As used herein the terms "enzymatically active fragment" and "A chain" are intended to include such similar acting natural proteins. Examples of such A chains are diphtheria A chain, exotoxin A chain (from Pseudmonas aeruginosa), ricin A chain, abrin A chain, modeccin A chain, alpha-sarcin, Aleurites fordii proteins, dianthin proteins, Phytolacca americana proteins (PAP I, PAP II, and PAP-S), momordin, curcin, crotin, gelonin, mitogellin, restrictocin, phenomycin, and enomycin.

The derivation of an A chain from a whole natural toxin molecule involves breaking the bond(s) between the A and B chain(s) (eg, reducing the disulfide bonds(s) between the A and B chain(s) with an appropriate reducing agent, such as 2-mercaptoethanol or dithiothreitol) and isolating the A chain from the B chain(s). The isolation may be carried out chromatographically or by other conventional protein fractionation techniques. The natural proteins that have enzymatic activity similar to the A chains of the natural protein toxins may be isolated from their sources, typically plant parts, by conventional protein extraction and isolation techniques. Alternatively, and preferably, these amino acid sequences are derived using recombinant techniques.

B.2. The Intracellular Cleavage Site

The intracellular cleavage site domain of the cytotoxin is preferably one that functions in a way that mimics the manner in which a natural toxin liberates its A chain. Three cleavage mechanisms are postulated currently: (1) proteolysis either enzymatic or chemical (eg, pH change), (2) disulfide reduction, and most commonly (3) a combination of (1) and (2). The cleavage site(s) of such domains is substantially stable extracellularly and is labile intracellularly. In the cleavage mechanisms involving proteolysis and disulfide reduction, extracellular stability is probably due to the position of the disulfide cleavage site in the extracellular tertiary structure of the conjugate. That is, the site is not exposed to cleavage agents in the extracellular environment, but is exposed in the intracellular environment due to a change in the tertiary structure of the molecule. Cleavage sites whose lability depends on pH are stable in extracellular environments, eg, blood, having a substantially neutral pH. The lower pH of certain intracellular compartments (eg, within a lysosome or receptosome) makes the site labile. The cleavage site domain comprises a sequence of amino acids that includes residues that are susceptible to proteolysis such as by lysosomal proteases. When disulfide reduction is involved the sequence will obviously contain a disulfide bridge

formed by spaced cysteine residues. When both proteolysis and reduction are involved the A chain is liberated from the remainder of the toxin conjugate by proteolysis at the sensitive residues that causes a nick or break in the polypeptide backbone of the molecule and reduction of the disulfide bond. Examples of residues that are lysosomal protease sensitive are arginine, lysine, phenylalanine, tyrosine, and tryptophan.

In a preferred embodiment, this cleavage site in a serum stable domain is proximate the enzymatically active domain and forms an extension of the C-terminus thereof. Suoh a configuration can be provided most conveniently by providing an extended "A" fragment from a naturally occurring toxin into a portion of the natural B chain. Recombinant techniques are best suited to this embodiment, since convenient peptide cleavage techniques specific for the desired extension may not exist for a given toxin. However, the coding sequence can be cleaved and modified so as to encode for just the desired fragment.

B.3. The Translocation Domain

The internalization facilitating or "translocation" domain of the conjugate participates in interacting with the cell or vesicle wall whereby the wall is penetrated, opened, or disrupted to enable the conjugate to reach the intracellular compartment. The internalization facilitating domain may be identical to, or substantially similar in, amino acid content and sequence to an internalization facilitating domain of a bacterial or plant toxin or a polypeptide that is known to interact similarly with cell membranes. In the case of DT, the domain has been identified as being "hydrophobic" and located within the B fragment. The sequence of that segment possibly includes the following:

Val-Ala-Gln-Ala-Ile-Pro-Leu-Val-Gly-Glu-Leu
Val-Asp-Ile-Gly-Phe-Ala-Ala-Tyr-Asn-Phe-Val
Glu-Ser-Ile-Ile-Asn-Leu-Phe-Gln-Val-Val

Examples of polypeptides that are known to have a similar interaction with cell membranes are melittin:

Gly-Ile-Gly-Ala-Val-Leu-Lys-Val-Leu-Thr-Thr-Gly-Leu-
Pro-Ala-Leu-Ile-Ser-Trp-Ile-Lys-Arg-Lys-Arg-Gln-Gln

and delta lysine:

Met-Ala-Gln-Asp-Ile-Ile-Ser-Thr-Ile-Gly-Asp-Leu-Val-
Lys-Trp-Ile-Ile-Asp-Thr-Val-Asn-Lys-Phe-Thr-Lys-Lys.

The position of the domain in the toxin conjugate molecule may vary. It will usually be located adjacent to the carboxy terminus of the A chain but may also be located adjacent to the amino terminus of the A chain. More than one internalization facilitating domain may be included in the conjugate if desired.

Since this domain is positioned adjacent the A chain, its inclusion in the toxin conjugate is most conveniently accomplished by recombinant DNA techniques. In DT, for example, the extension of the polypeptide sequence at the C terminus approximately 193 amino acids into the B chain provides such an internalization domain. Therefore, cloning and expression of the coding sequence for this portion of the DT toxin would provide the desired configuration. Alternatively, the oligonucleotides encoding known internalizing domains such as those exemplified above can be ligated to the nucleotides encoding the desired A fragment for cloning and expression.

B.4. The Spacer

The spacer comprises a sequence of amino acids that can be described in one of three non-mutually exclusive ways. In any case, the end of the spacer that attaches to the binding moiety may have a "reactive amino acid" residue at or near the terminus that provides a site for conjugating the binding moiety. For

instance, if a reactive amino group at or near the end of the spacer is desired one or more lysine residues may be located near one terminus of the spacer fragment. If a reactive sulfhydryl group is desired, a cysteine residue may be situated similarly.

In one aspect, the spacer is described as having one or more extended structure portions (segments in which the peptide bond angles are enlarged) linked by segments that are flexible. Each end of the spacer terminates with a flexible segment. The spacer thus links the target cell binding moiety to the remainder of the molecule with the extended structure portion(s) serving to separate the binding moiety and the remainder of the molecule and the flexible segments permitting three dimensional movement of the binding moiety and the remainder of the molecule. Thus the spacer can be described as being formed from a series of extended structure portions in tandem with intermediate flexible regions (the flexible regions lie on either side of the extended structure regions), ie, has the general formula $(\text{flex-rigid})_m$ flex. The extended (rigid) portion(s) of the spacer is preferably formed of a series of 4 to 8 prolines while the flexible portions are preferably composed of 4-8 amino acid residues each selected individually from the group consisting of serine, glycine, or threonine. The series of prolines form a left-handed proline II helix. These preferred spacers (less terminal reactive residue) may be represented by the formula

$(F\text{-}(Pro)_n)_mF$

wherein F represents a flexible sequence composed of amino acids each selected independently from the group consisting of serine, glycine, or threonine, n is an integer from 4 to 8 inclusive, and m is an integer from 1 to 4 inclusive. The flexible sequences may be the same or different. A particularly preferred spacer domain (less terminal reactive residue) is defined by the sequence

Gly-Thr-Gly-Ser-Gly-$(Pro)_n$-Ser-Gly-Ser-Gly-Thr where n is an integer from 4 to 8, inclusive, most preferably 6. A particularly preferred terminal reactive residue is cys.

In a second aspect, the spacer is substantially nonhydrophobic so that it has a neutral or positive effect on the water solubility of the conjugate. The spacer's hydrophobicity may be determined by summing the hydrophobicities of the individual amino acids (measured by partition coefficient tests) of which it is composed. A substantially nonhydrophobic sequence will measure neutral or hydrophilic. The hydrophilic nature of this segment will also place it on the surface of the configured molecule, thereby permitting accessibility for conjugation.

If the function of a particular spacer is merely to provide solubility during processing to a cytotoxic portion amino acid sequence, this property is, indeed, the only property required of it. Generally, the spacer is a relatively hydrophilic sequence, typically containing a cysteine residue.

In a third aspect, the spacer can also be described in functional terms as substantially stable in human serum, having a length selected such that it provides an extended structure link at least about 15 A long, preferably about 30 to about 100 A long, between the binding moiety and the remainder of the conjugate molecule, as being substantially non-hydrophobic so as not to adversely affect solubility, and having sufficient flexibility to permit three dimensional movement of the cytotoxic component with respect to the binding component.

## B.5. The Target Cell Binding Moiety

The binding moiety may be any ligand that has the required target cell specificity. Antibodies, particularly monoclonal antibodies or their antigen binding fragments, are preferred binding moieties. Monoclonal antibodies against surface receptors of target cells may be made by the somatic cell hybridization procedure first described by Kohler, G. and Milstein, C., Nature, (1975) 256:495-497. The cell lines, reagents, and conditions used in this procedure are well known and have been reviewed extensively in the literature (Somatic Cell Genetics, (1979) 5:957-972). Briefly the procedure involves immunizing a host with the immunogen of interest, collecting antibody-producing cells from the immunized host, fusing the antibody-producing cells from the immunized host with an appropriate tumor cell line using a fusogen such as polyethylene glycol, growing the cells in a selective medium to eliminate unhybridized partners, identifying hybridomas that produce antibody against the immunogen, growing such hybridomas, and collecting monoclonal antibodies from the resulting culture medium (or body fluid when grown in vivo). Antigen binding fragments (Fab, Fab', $F(ab')_2$, Fv) of the monoclonal antibodies may be made by digesting the whole Ig with an appropriate protease, for instance papain in the case of Fab and pepsin in the case of $F(ab')_2$. Antigen binding fragments will be particularly useful in instances where it is desired that the binding moiety lack its natural effector function. Antibodies of current interest will typically be of human, rat or murine origin since rat, mouse and human tumor cell lines are available for fusion. Also, a variety of

antitumor monoclonal antibody reagents are rapidly becoming available. Human monoclonal antibodies are preferred for use in making conjugates for use in human therapy because of the reduced likelihood of immunogenicity.

## C. Methods of Preparation

### C.1 The Non-binding Portion (Cytotoxic-spacer Portions)

Depending on the sizes of the four polypeptide domains that make up the nonbinding portion of the conjugate toxins, these domains may be synthesized individually or as subunits by conventional polypeptide synthesis techniques (Margolin, A. and Merrifield, R.B., Ann Rev Biochem, (1970) 39:841) and combined in sequence by known procedures.

Recombinant DNA methodology provides an alternative and preferred way of synthesizing the nonbinding portion of the conjugate, either as individual subunits, or as an entire fused polypeptide. This process involves obtaining a DNA sequence that encodes the nonbinding portion (or a particular domain or combination of domains) inserting the DNA sequence into a suitable expression vector, transforming microorganisms or cells with the vector, growing transformants that produce the desired fragment and harvesting the desired fragment from the transformants or their growth medium. The coding sequence may be made by synthesis of DNA subunits that encode portions of the enzymatically active fragment, translocation domain, cleavage site domain, and spacer domain and assembling them by ligation techniques known in the art, or by cloning those portions of naturally occuring genes which encode the desired protein. The DNA sequence that encodes the enzymatically active fragment will normally be isolated from naturally occurring DNA; as may the sequence encoding the cleavage and translocation domains. These and the spacer encoding sequence may also be made by conventional DNA synthesis techniques, and may be reproduced by conventional DNA cloning methods. Partial structural genes of bacterial or plant toxins that lack a binding function but retain their enzymatic, cleavage and internalization functions (eg, the CRM 45 mutant of DT) may be cloned and ligated to a DNA sequence that encodes the spacer. The ligation product is inserted into suitable expression hosts and expressed to make the nonbinding portion of the conjugate.

### C.2. The Binding Portions

The binding portions of the conjugates of the invention are antibodies or fragments thereof. Use of monoclonal antibodies is preferred. Antibodies are prepared by means well known in the art, and can be isolated from serum, from spleen, or most preferably prepared and isolated from antibody secreting hybridomas. Many are commercially available (see B.5 herein).

### C.3. Conjugation of Binding and Non-binding Portions

Since the antibodies or fragments thereof are prepared by reactions of immunoglobulins isolated from serum or from hydridomas, they must be linked in vitro to the non-binding domains. In a preferred configuration of the non-binding fragment, the cytotoxic portion of the molecule is extended from its C terminus by a sequence providing a spacer which has been provided with a reactive amino acid residue, cysteine, located at or near its C terminus. While bifunctional reagents, such as SPDP, that result in a labile disulfide (C-S-S-C) bridge between the spacer and binding moiety may be used as coupling agents, (and have often been so used where a cleavable link was desired) bifunctional reagents that form a stable (nonreducible) bond, such as a thioether (C-S-C) link are preferred. Such bonds are not susceptible to cleavage in the environment of use, that is, they are not cleaved extracellularly in vivo. Several protein coupling agents that form thioether bonds are available. These agents usually contain on one end of the molecule an activated carboxyl group that will react with free amino groups, eg, an $\epsilon$-amino of a lysine of one of the conjugation partners (in this case the binding portion) and a maleimido or haloacetyl group that will react with a sulfhydryl of the other partner (in this case the non-binding portion) on the other end. Examples of such thioether-forming agents are active esters of the following acids: 6-maleimidocaproic acid, 2-bromoacetic acid, 2-iodoacetic acid,

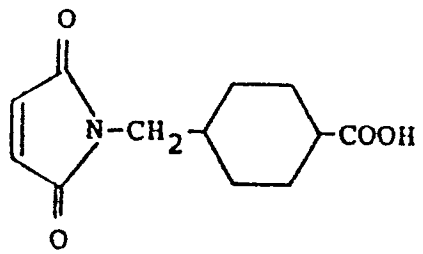 , 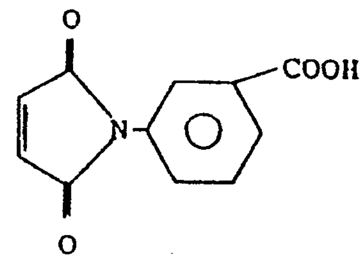 , and

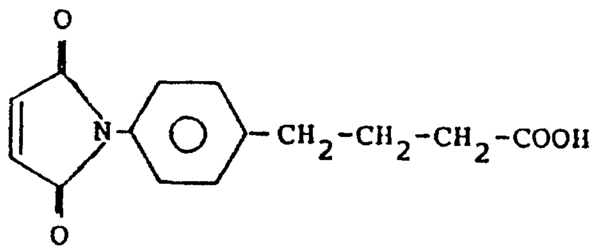

Activation of the carboxyl groups is required to permit reaction with amino groups of the chosen protein. The desired activated derivatives of the foregoing acids include most preferably the succinimidyl ester, ie

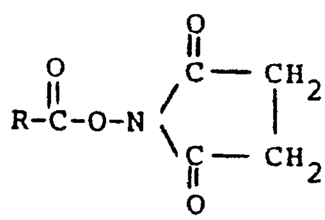

although others, especially esters, have been used, such as the water soluble ester formed from 1-hydroxy-2-nitro-4-sulfonic acid sodium salt,

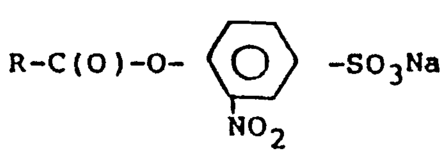

Other coupling agents that may be used are various bifunctional derivatives of imidoesters such as dimethyl adipimidate•HCl, active esters such as disuccinimidyl suberate, aldehydes such as glutaraldehyde, bis-azido compounds such as bis(p-azidobenzoyl)hexanediamine, bis-diazonium derivatives such as bis-(p-diazoziumbenzoyl)-ethylene diamine, diisocyanates such as tolylene 2,6-diisocyanate, and bis-active fluorine compounds such as 1,5-difluoro-2,4-dinitrobenzene. Heterologous permutations of such bifunctional derivatives may also be used as well as peptide bond-generating reagents such as carbodiimides.

In a typical, preferred approach, a thioether linkage is formed between a sulfhydryl on the non-binding portion of the conjugate and the coupling agent and an amide linkage is formed between an $\epsilon$-$NH_2$ of a lysine contained in the binding portion and the carboxyl of the coupling agent.

D. Mode of Administration

When used to kill target cells in vitro the conjugates will typically be added to the target cell culture medium in amounts ranging from about 1000 to about 100,000 conjugate molecules per target cell. The

formulation and mode of administration for in vitro use are not critical. Aqueous formulations that are compatible with the culture or perfusion medium will normally be used.

When used in vivo for prophylaxis or therapy of humans or animals (eg, farm, laboratory, sport or pet animals) the cytotoxins are administered to the patient in a manner and dose that induce the desired target cell reduction response. They will normally be administered parenterally, preferably intravenously. The dose and dosage regimen will depend upon the nature of the target cell and its population, the characteristics of the particular cytotoxin, eg its therapeutic index, the patient, and the patient's history. The amount of cytotoxin administered will typically be in the range of about 0.01 to about 1 mg/kg of patient weight.

For parenteral administration the cytotoxins will be formulated in a unit dosage injectable form (solution, suspension, emulsion) in association with a pharmaceutically acceptable parenteral vehicle. Such vehicles are inherently nontoxic and nontherapeutic. Examples of such vehicles are water, saline, Ringer's solution, dextrose solution, and Hank's solution. Nonaqueous vehicles such as fixed oils and ethyl oleate may also be used. Liposomes may be used as carriers. The vehicle may contain minor amounts of additives such as substances that enhance isotonicity and chemical stability, eg buffers and preservatives. The cytotoxin will typically be formulated in such vehicles at concentrations of about 1 mg/ml to 10 mg/ml.

E.1. Hosts and Control Sequences

Procaryotes may be used for both cloning and expression and most frequently are represented by various strains of E. coli. However, other microbial strains may also be used, such as bacilli, for example Bacillus subtilis, various species of Pseudomonas, or other bacterial strains. In such procaryotic systems, plasmid vectors which contain replication sites and control sequences derived from a species compatible with the host are used. For example, E. coli is typically transformed using derivatives of pBR322, a plasmid derived from an E. coli species by Bolivar, et al, Gene (1977) 2:95. pBR322 contains genes for ampicillin and tetracycline resistance, and thus provides additional markers which can be either retained or destroyed in constructing the desired vector. Commonly used procaryotic control sequences which are defined herein to include promoters for transcription initiation, optionally with an operator, along with ribosome binding site sequences, include such commonly used promoters as the beta-lactamase (penicillinase) and lactose (lac) promoter systems (Chang. et al, Nature (1977) 198:1056) and the tryptophan (trp) promoter system (Goeddel, et al Nucleic Acids Res (1980) 8:4057) and the lambda derived $P_L$ promoter and N-gene ribosome binding site (Shimatake, et al, Nature (1981) 292:128).

In addition to bacteria, eucaryotic microbes, such as yeast, may also be used as hosts. Laboratory strains of Saccharomyces cerevisiae, Baker's yeast, are most used although a number of other strains are commonly available. While vectors employing the 2 micron origin of replication are illustrated, Broach, J. R., Meth Enz (1983) 101:307, other plasmid vectors suitable for yeast expression are known (see, for example, Stinchcomb, et al, Nature (1979) 282:39, Tschempe, et al, Gene (1980) 10:157 and Clarke, L, et al, Meth Enz (1983) 101:300). Control sequences for yeast vectors include promoters for the synthesis of glycolytic enzymes (Hess, et al, J Adv Enzyme Reg (1968) 7:149: Holland, et al, Biochemistry (1978) 17:4900). Additional promoters known in the art include the promoter for 3-phosphoglycerate kinase (Hitzeman, et al, J Biol Chem (1980) 255:2073), and those for other glycolytic enzymes, such as glyceraldehyde-3-phosphate dehydrogenase, hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase. Other promoters, which have the additional advantage of transcription controlled by growth conditions are the promoter regions for alcohol dehydrogenase 2, isocytochrome C, acid phosphatase, degradative enzymes associated with nitrogen metabolism, and enzymes responsible for maltose and galactose utilization (Holland, ibid). It is also believed terminator sequences are desirable at the 3' end of the coding sequences. Such terminators are found in the 3' untranslated region following the coding sequences in yeast-derived genes. Many of the vectors illustrated contain control sequences derived from the enolase gene containing plasmid peno46 (Holland, M. J., et al, J Biol Chem (1981) 256:1385) or the LEU2 gene obtained from YEp13 (Broach, J., et al, Gene (1978) 8:121), however any vector containing a yeast compatible promoter, origin of replication and other control sequences is suitable.

It is also, of course, possible to express genes encoding polypeptides in eucaryotic host cell cultures derived from multicellular organisms. See, for example, Tissue Cultures, Academic Press, Cruz and Patterson, editors (1973). Useful host cell lines include VERO, HeLa cells, and Chinese hamster ovary (CHO) cells. Expression vectors for such cells ordinarily include promoters and control sequences compatible with mammalian cells such as, for example, the commonly used early and late promoters from Simian Virus 40 (SV 40) (Fiers, et al, Nature (1978) 273:113), or other viral promoters such as those derived from polyoma, Adenovirus 2, bovine papiloma virus, or avian sarcoma viruses. General aspects of mammalian

cell host system transformations have been described by Axel; U.S. Patent No. 4,399,216 issued 16 August 1983. It now appears, also that "enhancer" regions are important in optimizing expression; these are, generally, sequences found upstream or downstream of the promoter region in non-coding DNA regions. Origins of replication may be obtained, if needed, from viral sources. However, integration into the chromosome is a common mechanism for DNA replication in eucaryotes.

Finally, cells from and portions of higher plants have been found useful as recombinant hosts, and appropriate control sequences are available for expression in these systems. A suitable promoter and polyadenylation signal are those of the nopaline synthase (NOS) gene derived from the 3.2 kilobase (kb) HindIII-23 DNA fragment in the T-DNA region of A. tumefaciens Ti plasmid pTiT37 (Bevan, M., et al, Nucleic Acid Res (1983) 11:369; Depicker, A., et al, J Mol Appl Genet (1982) 1:5613). Suitable plant cells include cells derived from, or seedlings of, tobacco, petunia, and cotton. Other promoters include the maize promoter for alcohol dehydrogenase-1 or alcohol dehydrogenase-2 (Gerlach, W., L., et al, Proc Natl Acad Sci (USA) (1982) 79:2981), cauliflower mosaic virus promoter (Daubert, S., et al, Virology (1982) 122:444), and wheat promoter associated with the small subunit of ribulose biphosphate carboxylase (Broglie, R., et al, Biotechnology (1983) 1:55). Other polyadenylation signals which are available presently include those found on any of the above genes, or those of Schuler, et al, (Schuler, J. S., et al, Nucleic Acid Res (1982) 10:8225).

E.2. Transformations

Depending on the host cell used, transformation is done using standard techniques appropriate to such cells. The calcium treatment employing calcium chloride, as described by Cohen, S. N., Proc Natl Acad Sci (USA) (1972) 69:2110, or the $RbCl_2$ method described in Maniatis, et al, Molecular Cloning: A Laboratory Manual (1982) Cold Spring Harbor Press, p. 254 was used for procaryotes or other cells which contain substantial cell wall barriers. Infection with Agrobacterium tumefaciens (Shaw, C. H., et al, Gene (1983) 23:315) is used for certain plant cells. For mammalian cells without such cell walls, the calcium phosphate precipitation method of Graham and van der Eb, Virology (1978) 52:546 is preferred. Transformations into yeast are carried out according to the method of Van Solingen, P., et al, J Bact (1977) 130:946 and Hsiao, C. L., et al, Proc Natl Acad Sci (USA) (1979) 76:3829.

E.3. Probing cDNA or Genomic Libraries

cDNA or genomic libraries are screened using the colony hybridization procedure. Each microtiter plate is replicated onto duplicate nitrocellulose filter papers (S & S type BA-85) and colonies are allowed to grow at 37° C for 14-16 hr on L agar containing 50 µg/ml Amp. The colonies are lysed and DNA fixed to the filter by sequential treatment for 5 min with 500 mM NaOH, 1.5 M NaCl, and are washed twice for 5 min each time with 5 x standard saline citrate (SSC). Filters are air dried and baked at 80° C for 2 hr. The duplicate filters are prehybridized at 42° C for 6-8 hr with 10 ml per filter of DNA hybridization buffer (5 x SSC, pH 7.0 5x Denhardt's solution (polyvinylpyrrolidine, plus Ficoll and bovine serum albumin: 1 x = 0.02% of each), 50 mM sodium phosphate buffer at pH 7.0, 0.2% SDS, 20 µg/ml Poly U, and 50 µg/ml denatured salmon sperm DNA).

The samples are hybridized with kinased probe under conditions which depend on the stringency desired. Typical moderately stringent conditions employ a temperature of 42° C for 24-36 hr with 1-5 ml/filter of DNA hybridization buffer containing probe. For higher stringencies high temperatures and shorter times are employed. The filters are washed four times for 30 min each time at 37° C with 2 x SSC, 0.2% SDS and 50 mM sodium phosphate buffer at pH 7, then are washed twice with 2 x SSC and 0.2% SDS, air dried, and are autoradiographed at -70° C for 2 to 3 days.

E.4. Vector Construction

Construction of suitable vectors containing the desired coding and control sequences employs standard ligation and restriction techniques which are well understood in the art. Isolated plasmids, DNA sequences, or synthesized oligonucleotides are cleaved, tailored, and religated in the form desired.

Site specific DNA cleavage is performed by treating with the suitable restriction enzyme (or enzymes) under conditions which are generally understood in the art, and the particulars of which are specified by the manufacturer of these commercially available restriction enzymes. See, e.g., New England Biolabs, Product Catalog. In general, about 1 µg of plasmid or DNA sequence is cleaved by one unit of enzyme in about 20 µl of buffer solution; in the examples herein, typically, an excess of restriction enzyme is used to insure

complete digestion of the DNA substrate. Incubation times of about one hour to two hours at about 37°C are workable, although variations can be tolerated. After each incubation, protein is removed by extraction with phenol/chloroform, and may be followed by ether extraction, and the nucleic acid recovered from aqueous fractions by precipitation with ethanol followed by running over a Sephadex G-50 spin column. If desired, size separation of the cleaved fragments may be performed by polyacrylamide gel or agarose gel electrophoresis using standard techniques. A general description of size separations is found in Methods in Enzymology (1980) 65:499-560.

Restriction cleaved fragments may be blunt ended by treating with the large fragment of E. coli DNA polymerase I (Klenow) in the presence of the four deoxynucleotide triphosphates (dNTPs) using incubation times of about 15 to 25 min at 20 to 25°C in 50 mM Tris pH 7.6, 50 mM NaCl, 6 mM $MgCl_2$, 6 mM DTT and 5-10 µM dNTPs. The Klenow fragment fills in at 5' sticky ends but chews back protruding 3' single strands, even though the four dNTPs are present. If desired, selective repair can be performed by supplying only one of the, or selected, dNTPs within the limitations dictated by the nature of the sticky ends. After treatment with Klenow, the mixture is extracted with phenol/chloroform and ethanol precipitated followed by running over a Sephadex G-50 spin column. Treatment under appropriate conditions with S1 nuclease results in hydrolysis of any single-stranded portion.

Exonuclease III attacks double-stranded DNA, but hydrolyzes beginning at the 3' end of the nucleotide sequence. Thus, digestion of a double-stranded DNA results in two 5' protruding sticky ends. Hydrolysis is carried out in a buffer containing 15 mM Tris, pH 8, 10 mM NaCl, 1 mM $MgCl_2$, and 0.1 mM DTT, using approximately 2000 units per µl exonuclease III. Ordinarily, 150 units of exonuclease III were used to react with 10 µg DNA.

Synthetic oligonucleotides are prepared by the triester method of Matteucci, et al (J Am Chem Soc - (1981) 103:3185) or using commercially available automated oligonucleotide synthesizers. Kinasing of single strands prior to annealing or for labeling is achieved using an excess, e.g., approximately 10 units of polynucleotide kinase to 0.1 nmole substrate in the presence of 50 mM Tris, pH 7.6, 10 mM $MgCl_2$, 5 mM dithiothreitol, 1-2 mM ATP, 1.7 pmoles γ32P-ATP (2.9 mCi/mmole), 0.1 mM spermidine, 0.1 mM EDTA.

Ligations are performed in 15-30 µl volumes under the following standard conditions and temperatures: 20 mM Tris-Cl pH 7.5, 10 mM $MgCl_2$, 10 mM DTT, 33 µg/ml BSA, 10 mM-50 mM NaCl, and either 40 µM ATP, 0.01-0.02 (Weiss) units T4 DNA ligase at 0°C (for "sticky end" ligation) or 1 mM ATP, 0.3-0.6 (Weiss) units T4 DNA ligase at 14°C (for "blunt end" ligation). Intermolecular "sticky end" ligations are usually performed at 33-100 µg/ml total DNA concentrations (5-100 nM total end concentration). Intermolecular blunt end ligations (usually employing a 10-30 fold molar excess of linkers) are performed at 1 µM total ends concentration.

In vector construction employing "vector fragments", the Vector fragment is commonly treated with bacterial alkaline phosphatase (BAP) in order to remove the 5' phosphate and prevent religation of the vector. BAP digestions are conducted at pH 8 in approximately 150 mM Tris, in the presence of $Na^+$ and $Mg^{2+}$ using about 1 unit of BAP per µg of vector at 60°C for about one hour. In order to recover the nucleic acid fragments, the preparation is extracted with phenol/chloroform and ethanol precipitated and desalted by application to a Sephadex G-50 spin column. Alternatively, religation can be prevented in vectors which have been double digested by additional restriction enzyme digestion of the unwanted fragments.

For portions of vectors derived from cDNA or genomic DNA which require sequence modifications, site specific primer directed mutagenesis is used. This is conducted using a primer synthetic oligonucleotide complementary to a single stranded phage DNA to be mutagenized except for limited mismatching, representing the desired mutation. Briefly, the synthetic oligonucleotide is used as a primer to direct synthesis of a strand complementary to the phage, and the resulting double-stranded DNA is transformed into a phage-supporting host bacterium. Cultures of the transformed bacteria are plated in top agar, permitting plaque formation from single cells which harbor the phage.

Theoretically, 50% of the new plaques will contain the phage having, as a single strand, the mutated form; 50% will have the original sequence. The resulting plaques are hybridized with kinased synthetic primer at a temperature which permits hybridization of an exact match, but at which the mismatches with the original strand are sufficient to prevent hybridization. Plaques which hybridize with the probe are then picked, cultured, and the DNA recovered.

In more detail, approximately one pmole of the phage single stranded DNA template is mixed with approximately 10 pmoles of the synthetic oligonucleotide primer in 15 µl of 10 mM Tris, 10 mM $MgCl_2$, 90 mM NaCl. The mixture is heated to 67° for 3-5 min and then to 42° for 30 min. The mixture is then cooled on ice, and a cold solution containing the 4 dNTPs at 500 µM and 3-5 units of Polymerase I (Klenow) in sufficient buffer to bring the volume to 20-25 µl is added. The mixture is left at 0°C for 5 min and then brought to 37° for 30 min. The Klenow is then inactivated for 15 min at 75°, and the mixture transformed

into an appropriate host, such as E. coli JM103 or E. coli JM105 using 1 µl reaction mixture per 300 µl cells, which are grown on yeast extract-typtone agar plates. The resulting phage plaques are transferred to filters by lifting onto nitrocellulose, and pre-hybridized in 5 ml/filter of 6 x SSC, 5 x Denhardt's, 0.1% SDS, 50 µg/ml carrier (yeast RNA salmon sperm DNA etc.) at the desired temperature for 1-2 hr.

The fixed, pre-hybridized filters are then hybridized with 2 x $10^5$ cpm/ml of kinased synthetic primer oligonucleotide (approximately 2-10 x $10^7$ cpm/µg) for 3-16 hr, and then washed in 6 x SSC once at room temperature for 5 min and then at the appropriate stringent temperature for 5 min. A simultaneous control run containing the original phage is used to verify that hybridization does not take place to the non-mutagenized strands.

E.5. Verification of Construction

In the constructions set forth below, correct ligations for plasmid construction are confirmed by first transforming E. coli strain MM294 obtained from E. coli Genetic Stock Center, CGSC #6135, or other suitable host with the ligation mixture. Successful transformants are selected by ampicillin, tetracycline or other antibiotic resistance or using other markers depending on the mode of plasmid construction, as is understood in the art. Plasmids from the transformants are then prepared according to the method of Clewell, D. B., et al, Proc Natl Acad Sci (USA) (1969) 62:1159, optionally following chloramphenicol amplification (Clewell, D. B., J Bacteriol (1972) 110:667). The isolated DNA is analyzed by restriction and/or sequenced by the dideoxy method of Sanger, F., et al, Proc Natl Acad Sci (USA) (1977) 74:5463 as further described by Messing, et al, Nucleic Acids Res (1981) 9:309, or by the method of Maxam, et al, Methods in Enzymology (1980) 65:499.

E.6. Hosts Exemplified

Host strains used in cloning and expression herein are as follows:

For cloning and sequencing, and for expression of construction under control of most bacterial promoters, E. coli (K12) strain MM294 (E. coli Genetic Stock Center CGSC #6135), Talmadge, K., et al, Gene (1980) 12:235; Meselson, M., et al, Nature (1968) 217:1110, was used as the host. For expression under control of the $P_LN_{RBS}$ promoter, E. coli strain K12 MC1000 lambda lysogen, $N_7N_{53}cI857SusP_{80}$, ATCC 39531 (hereinafter sometimes referred to as MC1000-39531) is used.

For M13 phage recombinants, E. coli strains susceptible to phage infection, such as E. coli K12 strain DG98, JM103 or JM105 are employed. The DG98 strain has been deposited with ATCC July 13, 1984 and has accession number 39768.

E.7. Construction of Host Vectors for Expression

The successful expression attained by the invention depends upon correct utilization of the suitable control sequences to regulate expression of the desired toxin fragment. Therefore, whatever the host, control sequences compatible with and suitable for that host are positioned operably with respect to the coding sequence, using a properly placed "start" codon at the 5' end of the desired sequence. Any "native" control sequences are eliminated. The vectors of the invention place the coding sequence for the desired protein immediately preceded by an ATG start codon directly downstream from control systems chosen to be compatible with the particular host.

It is also important, in obtaining good production of the desired fragments, to regulate the "time" of production so as to minimize any lethal effect on the host cell. Most typically, even for procaryotes, this is done by delaying expression of the desired protein sequences until substantial growth has occurred. Accordingly, it is desirable to utilize control sequences which are subject to environmental conditions. By maintaining conditions that repress expression during growth phase, and then converting to conditions which permit expression at the desired time, the negative aspects of any potentially lethal effect can be minimized.

In two particularly preferred approaches, these regulatable control sequences are compatible with procaryotic hosts. The trp promoter is a regulatable promoter where expression of the operably linked sequence can be controlled by the level of tryptophan in the medium. By maintaining high tryptophan levels during growth, expression is repressed. Depletion or competitive inhibition of tryptophan turns on the promoter and permits expression.

Still more preferred is the $P_L$ promoter derived from lambda phage. This promoter is regulated by a protein which can be temperature sensitive. (There are mutant forms of the wild type repressor, e.g., $CI_{857}$

which have this characteristic known in the art.) When used in a host which is able to synthesize this mutant form of repressor (such as E. coli K12 strain MC1000 lysogenic for the lambda phage $N_7N_{53}CI_{857}SusP_{80}$), the $P_L$ promoter will be switched on when the temperature is raised because the higher temperature inactivates the mutant CI repressor. Thus, the host cells can be grown at low temperature without, or with, low production of the foreign protein. The temperature is then raised when growth has been attained and desired protein production is desired.

A plasmid which has temperature sensitive copy number control may also be applied. If the cells are grown at low temperatures, coding sequences contained in the plasmid are replicated at low levels; at higher temperatures, the number of such copies is increased. The amount of protein produced is thus indirectly managed by regulating the number of available copies of its coding sequence.

Several vectors which provide control sequences and replicons useful in obtaining the various conjugate non-binding sequences are described below. These include: pCS3, a temperature-sensitive, high copy number plasmid, pFC5, $pP_L322$, and $pP_LKan$, which are sources of the $P_LN_{RBS}$ control sequences, pPLOP, which contains both this cassette and the replicon sequences of pCS3, pTRP3 which has the trp control sequences available as an EcoRI/HindIII cassette, and pDG141 which contains this cassette, as well as an alternate control sequence wherein the trp control sequences are followed by an ATG start codon immediately upstream from a SacI site.

E.7.a. Construction of pCS3

The temperature-sensitive, high copy number plasmid pCS3 was deposited with ATCC 3 June 1982 and has accession number 39142.

pCS3 is derived from pEW27 and pOP9. pEW27 is described by E. M. Wong, Proc Natl Acad Sci (USA) (1982) 79:3570. It contains mutations near its origin of replication which provide for temperature regulation of copy number. As a result of these mutations replication occurs in high copy number at high temperatures, but at low copy number at lower temperatures.

pOP9 is a high copy number plasmid at all temperatures which was constructed by inserting into pBR322 the EcoRI/PvuII origin containing fragment from Col E1 type plasmid pOP6 (Gelfand, D., et al, Proc Natl Acad Sci (USA) (1978) 75:5869). Before insertion, this fragment was modified as follows: 50 µg of pOP6 was digested to completion with 20 units each BamHI and SstI. In order to eliminate the SstI 3' protruding ends and "fill in" the BamHI 5' ends, the digested pOP6 DNA was treated with E. coli DNA polymerase I (Klenow) in a two-stage reaction first at 20°C for elimination of the 3' SstI protruding end and then at 9°C for repair at the 5' end. The blunt-ended fragment was digested and 0.02 pmole used to transform competent DG75 (O'Farrell, P., et al, J Bacteriology (1978) 134:645-654). Transformants were selected on L plates containing 50 µg/ml ampicillin and screened for a 3.3 kb deletion, loss of an SstI site, and presence of a newly formed BamHI site.

One candidate, designated pOP7, was chosen and the BamHI site deleted by digesting 25 g of pOP7 with 20 units BamHI, repairing with E. coli DNA polymerase I fragment (Klenow), and religating with T4 DNA ligase. Competent DG75 was treated with 0.1 µg of the DNA and transformants selected on L plates containing 50 µg/ml ampicillin. Candidates were screened for the loss of the BamHI restriction site. pOP8 was selected. To obtain pOP9 the AvaI(repaired)/EcoRI $Tet^R$ fragment from pBR322 was prepared and isolated and ligated to the isolated PvuII(partial)/EcoRI 3560 bp fragment from pOP8.

Ligation of 1.42 kb EcoRI/AvaI(repair) $Tet^R$ (fragment A) and 3.56 kb EcoRI/PvuII $Amp^R$ (fragment B) used 0.5 µg of fragment B and 4.5 µg of fragment A in a two-stage reaction in order to favor intermolecular ligation of the EcoRI ends.

Competent DG75 was transformed with 5 µl of the ligation mixture, and transformants were selected on ampicillin (50 µg/ml) containing plates, pOP9, isolated from $Amp^R$ $Tet^R$ transformants showed high copy number, colicin resistance, single restriction sites for EcoRI, BamHI, PvuII, HindIII, 2 restriction sites for HincII, and the appropriate size and HaeIII digestion pattern.

To obtain pCS3, 50 µg pEW27 DNA was digested to completion with PvuII and the EcoRI. Similarly, 50 µg of pOP9 was digested to completion with PvuII and EcoRI and the 3.3 kb fragment was isolated.

0.36 µg (0.327 pmoles) pEW27 fragment and 0.35 µg (0.16 pmoles) pOP9 fragment were ligated and used to transform E. coli MM294. $Amp^RTet^R$ transformants were selected. Successful colonies were initially screened at 30°C and 41°C on beta-lactamase assay plate and then for plasmid DNA levels following growth at 30°C and 41°C. A successful candidate, designated pCS3, was confirmed by sequencing.

E.7.b.Vectors Supplying the $P_L$ Control Sequence

Three plasmids were constructed which can serve as sources for the EcoRI (or PstI)/HindIII $P_LN_{RBS}$ cassette. For each of these plasmids, the DNA sequence containing $P_L$ λ phage promoter and the ribosome binding site for the N-gene ($N_{RBS}$) is obtained from a derivative of pKC30 described by Shimatake and Rosenberg, Nature (1981) 292:128. pKC30 contains a 2.34 kb fragment from λ phage cloned into the HindIII/BamHI vector fragment from pBR322. The $P_L$ promoter and $N_{RBS}$ occupy a segment in pKC30 between a BglII and HpaI site. The derivative has the BglII site converted to an EcoRI site.

The BglII site immediately preceding the $P_L$ promoter was converted into an EcoRI site as follows: pKC30 was digested with BglII, repaired with Klenow and dNTPs and ligated with T4 ligase to an EcoRI linker (available from New England Biolabs) and transformed into E. coli K12 strain MM294 Lambda$^+$. Plasmids were isolated from $Amp^R$ $Tet^S$ transformants and the desired sequence confirmed by restriction analysis and sequencing. The resulting plasmid, pFC3, was double-digested with PvuI and HpaI to obtain an approximately 540 bp fragment framing the desired sequence. This fragment was partially digested with HinfI and the 424 bp fragment isolated and treated with Klenow and dATP, followed by S1 nuclease, to generate a blunt-ended fragment with the 3' terminal sequence -AGGAGAA, where the -AGGAGA portion is the $N_{RBS}$. This fragment was restricted with EcoRI to give a 347 base pair DNA fragment with 5'-EcoRI (sticky) and HinfI(partial repair S1 blunt)-3' termini.

Preparation of pFC5

pβI-Z15, deposited 13 January 1984, ATCC No. 39578, was prepared by fusing a sequence containing ATG plus 140 bp of β-IFN fused to lac-Z into pBR322. In pβI-Z15, the EcoRI site of pBR322 is retained, and the insert contains a HindIII site immediately preceding the ATG start codon of β-IFN. pβI-Z15 was restricted with HindIII, repaired with Klenow and dNTPs, and then digested with EcoRI. The resulting EcoRI/HindIII (repaired) vector fragment was ligated with the EcoRI/HinfI (repaired) fragment above, and the ligation mixture used to transform MC1000-39531. Transformants containing the successful construction were identified by ability to grow on lactose minimal plates at 34° but not at 30°. (Transformations were plated on X-gal-Amp plates at 30° and 34° and minimal-lactose plates at 30° and 34°. Transformants with the proper construction are blue on X-gal-Amp plates at both temperatures, but on minimal lactose plates, grow only at 34°.) The successful construct was designated pFC5.

Preparation of $pP_L322$

In the alternative, pBR322 may also be used as the cloning vector to carry the desired EcoRI/HindIII $P_L$-$N_{RBS}$ cassette. pBR322 was digested with HindIII, repaired with Klenow and dNTPs, and then further digested with EcoRI. The vector fragment was then ligated to the EcoRI/HinfI(repaired) fragment prepared above, and the ligation mixture transformed into MC1000-39531. Successful transformants were identified as $Amp^R$ $Tet^S$ colonies. Plasmids were isolated from successful transformants and a successful ligation was confirmed by sequencing, and designated $pP_L322$.

Preparation of $pP_LKan$

The third host plasmid vector used to obtain the cassette was pDG144, deposited January 13, 1984, ATCC No. 39579. pDG144 is extensively described in another application and is not part of the invention. It is an altered pBR322 containing an intact $Amp^R$ gene, and a coding sequence for a protein capable of conferring resistance to kanamycin ($Kan^R$). The $Kan^R$ coding sequence is preceded by a synthetic polylinker. Since pDG144 contains neither a promoter nor a ribosome binding site preceding the coding sequence, $Kan^R$ is not expressed, and cells harboring pDG144 are sensitive to Kan and to structurally related antibiotics. The polylinker sequence immediately preceding the ATG start codon for the kanamycin gene can be removed by digestion with EcoRI and HindIII and $P_LN_{RBS}$ inserted.

Accordingly, pDG144 was digested with HindIII, blunt-ended with Klenow and dNTPs, and then digested with EcoRI. The vector fragment was ligated with the above-prepared EcoRI/HinfI(repaired) fragment and transformed into MC1000-39531. $Amp^R$ $Kan^R$ colonies were selected, plasmids isolated and the correct sequence construction verified by restriction analysis and sequencing. One plasmid containing the correct sequence was designated $pP_LKan$.

Each of the above resulting vectors, pFC5, $pP_L322$, and $pP_LKan$, may be used to clone and provide the EcoRI/HindIII $P_LN_{RBS}$ cassette. The cassette can then conveniently be placed behind an ATG start codon which contains a HindIII site immediately preceding it.

E.7.c. Completion of pPLOP

pCS3 was then modified to provide the $P_L$ and $N_{RBS}$ control sequences. pCS3 was digested with HindIII, and then digested with EcoRI. The vector fragment was ligated with an isolated EcoRI/HindIII from pFC5 containing the $P_L N_{RBS}$ and transformed into E. coli MM294. The correct construction of isolated plasmid DNA was confirmed by restriction analysis and sequencing and the plasmid designated pPLOP. pPLOP was deposited with ATCC 18 December 1984 and has accession number 39947.

E.7.d.Construction of pTRP3

To construct the host vector containing the trp control sequences behind a HindIII site, the trp promoter/operator/ribosome binding site sequence, lacking the attenuator region, was obtained from pVH153, obtained from C. Yanofsky, Stanford University. Trp sequences are available in a variety of such plasmids known in the art. pVH153 was treated with HhaI (which cuts leaving an exposed 3' sticky end just 5' of the trp promoter) blunt-ended with Klenow, and partially digested with TaqI. The 99 bp fragment corresponding to restriction at the TaqI site, 6 nucleotides preceding the ATG start codon of trp leader was isolated, and then ligated to EcoRI (repair)/ClaI digested, pBR322 to provide pTRP3. pTRP3 was deposited with ATCC 18 December 1984 and has accession number 39946.

E.7.e.Construction of pDG141

pDG141 contains the trp control sequences immediately upstream from an ATG start codon. It was deposited with the ATCC January 24, 1984, and given the accession number 39588. The sequence downstream of the ATG provides a SacI cleavage site which cuts between the G and the succeeding bp. In the construction of pDG141, a derivative of pBR322, pTRP3, is used to provide a trp (PstI/HindIII) cassette and pBW20 to provide the ATG and SacI site.

Construction of pBW20

pBW20 contains a synthetic ATG-containing dodecamer cloned into the HindIII/PvuII vector fragment from pBR322. The dodecamer, TATGAGCTCATA, contains SstI (or SacI) sites. To prepare pBW20, pBR322 was digested with HindIII, repaired with Klenow and the four dNTPs, and then digested with PvuII. The vector fragment was ligated with the self-complementary dodecamer and transformed into E. coli MM294 and the correct construction confirmed by plasmid isolation and sequencing.

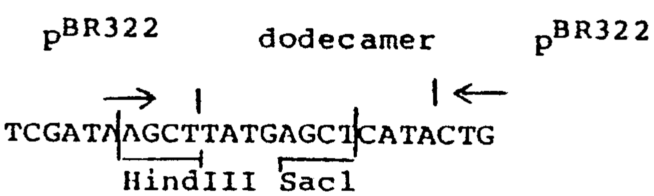


12 ng of pTRP3 restricted with PstI and HindIII was ligated with 1.34 ng of similarly restricted pBW20. The ligation mixture was subsequently digested with BamHI to linearize any ligation products which contained the HindIII/PstI unwanted vector fragment from pTRP3. The ligation mixture was used to transform E. coli MM294, and the desired colonies selected on plates of L-Broth containing 50 µg/ml ampicillin pre-spread with 500 µg tryptophan. Correct construction was confirmed by sequencing.

F. Diphtheria Toxin Components and Conjugates

An illustrative and preferred embodiment of the invention comprises the components and intermediates in a specific conjugated toxin. In this toxin, the binding fragment consists of anti-Daudi antibodies which are linked by reaction with the succinimidyl ester of m-maleimidobenzoic acid to a spacer portion of the formula

Gly-Thr-Gly-Ser-Gly$(Pro)_6$ Ser-Gly-Ser-Gly-Thr-Cys

spacer reactive amino acid

which is in turn a C terminal extension of a portion of the naturally occurring diphtheria toxin. Thus, the conjugate toxin here exemplified can be represented by the formula:

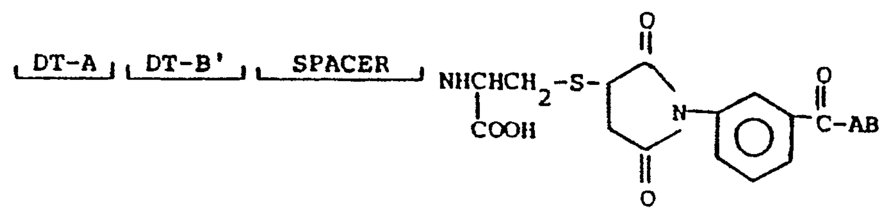

wherein DTA represents the enzymatically active portion, or "A" chain of diphtheria toxin, DT-B' represents the first approximately 190 amino acids of the diphtheria B chain, and AB represents the anti-Daudi antibody.

In this embodiment, three of the elements of the non-binding portion of the conjugate toxin are derived from diphtheria toxin; the enzymatically active domain, the cleavage site domain and the translocation domain. The mature diphtheria toxin molecule including both A and B chains contains 535 amino acid residues of which the A chain (the amino terminal fragment) contains 193 residues and the B chain (the carboxy terminal fragment) contains 342 residues. It appears that the intracellular cleavage site between the A and B chains in the native toxin is after one of the three Arg residues. Such cleavage readily takes place in vitro catalyzed by trypsin-like enzymes. It is thought that a similar cleavage takes place in vivo, thus exposing the disulfide link between the cysteine at position 186 and that at 201 for reductive intracellular cleavage. It is known that the amino terminal 193/342 of the B fragment contains sequences that cause the toxin to insert into artificial lipid bilayers under appropriate conditions forming ion conductive channels (Kagan, B.L., et al, Proc Natl Acad Sci (USA), (1981) 78:4950; Donovan, J.J., et al Proc Natl Acad Sci, (1972) 78:172 (1972); Kaiser, G., et al Biochem Biophys Res Commun, (1981) 99:358; FEBS Letters (1983) 160:82). Thus the portions of the diphtheria toxin which are embodied in the illustrative conjugate toxin contain the intracellular cleavage/extracellularly stable connection between the A and B chain and at least a portion of the hydrophobic domain responsible for translocation of the cytotoxic portion into the cytosol.

Fig. 1 shows the sequence of the diphtheria toxin gene and the flanking regions, along with the deduced amino acid sequence. The deduced sequence is in reasonable agreement with the previously reported primary amino acid sequence data (Delange, R.J., et al, Proc Natl Acad Sci (USA) (1976) 73:69; Delange, R.J., et al, J Bio Chem (1979) 254:5827; Drazin, R.E., et al, (ibid) 5832 (1979); Delange, R.J., et al, (ibid) 5838 (1979); Falmagne, P., et al, Biochim Biophys Acta (1978) 535:54; Falmagne, P., et al, Toxicon (1979) 17:supp 1 46; Lambotte, P., et al, J Cell Biol (1980) 87:837; Capiau, C., et al, Arch Phys (1982) 90:B-96; Falmagne, P., et al, Toxicon (1982) 20:243). The deduced sequence assumes a leader sequence as shown, consistent with the fact that DTB is secreted from the natural source, C. Diphtheriae and with the fact that the sequence in this region strongly resembles known signal peptides (Michaelis, S., et al, Ann Rev Microbiol (1982) 36:435). It is presently believed that the GTG codon at position -25 serves as a start codon and encodes methionine rather than the valine there shown.

The entire toxin gene sequence is carried by bacteriophage-$\beta$ and can be isolated from the phage by restriction with Xba 1 and EcoRI. A shorter MspI fragment within this sequence (see Fig 1) comprises most of the sequence used in the illustrative construct herein, this fragment results from MspI restriction about 300 bp preceding the first amino acid codon, and at the site shown at the codon encoding amino acid 382, approximately in the middle of the B fragment.

The construction of the illustrated conjugate toxin may be summarized as follows:

the Msp portion of the gene containing the codons for the A chain and approximately half of the B chain are ligated to synthetic DNA encoding the desired spacer with its reactive amino acid (cysteine) terminus. The resulting oligonucleotide is then modified to delete the promoter and ribosome binding regions as well as the codons for the leader sequence. It is then ligated into an operably linked position with respect to the

$P_L$ promoter and N-gene ribosome binding site in suitable expression vector, along with an ATG start codon immediately preceding the glycine residue at position 1. Bacteria transformed with this expression vector produced the entire non-binding region of the molecule. The transformed cells are sonicated and the non-binding portion recovered from the sonicate. The non-binding portion is then linked to the anti-Daudi antibody in vitro using an activated m-maleimidobenzoic ester as linker.

Additional non-binding fragments are also recombinantly derived as illustrated below, and used to obtain alternate conjugates.

F.1. Isolation and Cloning of the MspI Fragment from the DT Gene

DNA was isolated from corynephage $\beta^{Tox+}$ grown on Corynebacterum diphtheriae $C7^{(-)tox-}$. (The host and phage are obtainable from J. Collier, University of California, Los Angeles; see Tweten, R.K., et al, J Bacteriol (1983) 156:680.

To prepare DNA, high-titered $\beta$ phage stocks were prepared in "TYE'medium" (15 g/l bactotryptone, 10 g/l yeast extract, 5 g/l NaCl supplemented with 1 mM $CaCl_2$), by the method of Holmes, R.K., et al J Virology (1969) 38:586. Upon completion of lysis, debris was removed by centrifugation at 13,000 x g for 5 min, and NaCl added to 0.5 M, followed by PEG to 100 g/l, and the mixture was stirred overnight at 4° C. The phages were concentrated by centrifugation at 13,000 x g for 15 min and resuspended in 100 mM Tris HCl pH 7.5, 100 mM NaCl, 20 mM EDTA. Pronase was added to 1 mg/ml and the mixture was incubated at 37° C for 2 hr. After removal of PEG by addition of potassium phosphate (dibasic:monobasic/2:1) to 23% and centrifugation at 6,000 x g for 5 min, the lower phase was extracted with phenol, ethanol precipitated and the DNA purified by banding in a CsCl-EtBr gradient.

Approximately 500 $\mu$g of the phage DNA (MW = 22 x $10^6$ daltons) was treated with EcoRI and XbaI and the resulting mixture run on 1.7 liters 1% agarose gel at 90 volts for 35 hr. The XbaI/EcoRI fragment (1.5 x $10^6$ daltons) containing the toxin gene was cut out, run through a syringe, and electroeluted in 1/10 TBE for 4 hrs at 500 volts onto a spectropore dialysis membrane. The DNA was retrieved from the membrane using 0.25% SDS in 1/10 TBE, phenol extracted, ether extracted, and ethanol precipitated.

The resulting DNA was further restricted with MspI, the DNA resolved on 5% PAGE, and the two MspI fragments isolated by the crush and soak method. The large Msp fraction (see Fig 1) which contained control sequences, leader, A, and partial B sequences from the toxin was cloned by ligating approximately 5 ng of the fragment with 2 $\mu$g of ClaI-restricted, BAPed, pBR322. The ligation mixture was transformed into E.coli MM294, and the desired clones determined by isolation of plasmids, restriction analysis and sequencing. The desired cloning vector was designated pMsp. Although this cloning was accomplished as above, constructions to provide the non-binding portion were obtained using phage directly as the source of Msp fragment.

F.2. Synthesis and Cloning of Spacer Coding Sequence

A DNA fragment encoding the amino acid sequence

Gly-Thr-Gly-Ser-Gly-(Pro)$_6$-Ser-Gly-Ser-Gly-Thr-Cys

and flanked by sequences defining convenient restriction sites and a stop codon was designed and synthesized by conventional DNA synthesis procedures.

The sequence,

```
AG CTT CCA GGC ACT GGA TCT GGC-
           Gly Thr Gly Ser Gly-

CCG CCG CCA CCG CCG CCT TCT GGA TCC GGT ACC TGC TGA G
Pro Pro Pro Pro Pro Pro Ser Gly Ser Gly Thr Cys Stop
```

and its complement were prepared using the triester method of Matteucci (supra) and annealed and kinased to give the double stranded sequence:

```
P-AGCTTCCAGGC-----------ACCTGCTGAG
        AGGTCCG-----------TGGACGACTCAGCT-P

HindIII                              SalI
```

The annealed sequence was cloned as follows: pBR322 (25.72 μg) was restricted with SalI and HindIII, BAPed, phenol extracted and desalted over a one cc Sephadex G-50 column. The kinased annealed, double stranded spacer encoding sequence (0.2 pmoles) was ligated with 1 μg of the plasmid vector fragment, and the ligation mixture was used to transform E.coli strain MM294. $Amp^RTet^S$ colonies were screened for plasmid size. The desired plasmid, pSA1 was confirmed by restriction analysis and sequencing.

F.3. Cloning of Spacer onto MspI Fragment

The plasmid, pMspSA2 which contains the MspI fragment coding sequence ligated to the spacer coding sequence was contructed as outlined in Fig 2.

pSA1 plasmid DNA (77 μg) was restricted with SalI and ClaI, run on a 12% polyacrylamide gel and the fragment containing the spacer arm sequence isolated by the crush and soak method. One half the sample was further restricted with AluI to give "Fragment A". As shown in Fig 2, the Alu cleavage results in a blunt end 6 bp upstream from the glycine codon.

The large MspI fragment (10 ng) isolated from phage as in F.1 was blunt ended by filling in with Klenow fragment and dNTPs. The mixture was run over a Sephadex G-50 column, treated with HindIII, and re run over a 1 cc Sephadex G-50 column to give "fragment B". As seen from Fig 1. HindIII restriction deletes a portion of the DT control sequences, but probably leaves at least a portion of the promoter and ribosome binding site, and the entire leader sequence.

For the vector, 77 μg pSA1 was restricted with HindIII and SalI, treated with BAP, and the vector DNA fragment purified with a 1 cc Sephadex G-50 column to give "fragment C".

The ligation mixture consisted of 4 μg of fragment C, 3 ng of fragment B, and 20 ng of fragment A under standard ligation conditions. Following ligation overnight at 12° C, the mixture was transformed into E.coli strain MM294, and $Amp^RTet^S$ colonies screened for plasmid size. The desired construct was identified by restriction analysis and confirmed by Maxam-Gilbert sequencing. This plasmid, pMspSA2 contains, between the HindIII and SalI sites of pBR322, a portion of the DT control sequence, leader sequence, A fragment, B fragment through the codon for amino acid 382, and the spacer arm codons.

F.4. Deletion of the DT Promoter and Leader Sequences

The preparation of pATGMspSA is outlined in Fig 3.

pTrpSmlMbo (55μg) was double digested with AccI and ClaI and the short fragment spanning the ATG start codon and a portion of the A fragment isolated. (See Fig 4 for relevant sequences in pTrpSmlMbo and ¶F.4.a. below for its construction.)

A vector fragment was prepared by digesting 25 μg of pMspSA with ClaI, and treating with BAP. The missing portions of the Msp toxin fragment were supplied by a digest of 50 μg of pMspSA with AccI and ClaI and isolating the 764 bp fragment between these sites in the coding sequence.

A ligation mixture containing 250 ng of the ATG-partial A fragment from pTrpSmlMbo, 700 ng of the partial A- partial B fragment from pMspSA and 2 μg of the spacer-vector fragment from pMspSA was transformed into E.coli MM294 and $Amp^RTet^S$ colonies selected. The correct construction was confirmed by restriction analysis.

F.4.a. Construction of pTrpSmlMbo

pTrpSmlMbo contains the DT-A fragment coding sequence followed by the Mbo terminator sequence (supra) under the control of the trp promoter. The construction is from pTS12 (a plasmid containing the DT-A and Mbo terminator) and pDG141, which contains the trp promoter (see Fig 4)

To construct pTS12, the oligonucleotide

```
GA  TCT  GTT  GGC  TCG  AGT  TGA
Arg  Ser  Val  Gly  Ser  Ser  Term
```

which encodes the amino acid sequence subsequent to the Mbo cleavage site for six additional amino acids prior to a termination codon was synthesized using the triester method of Matteucci, et al (supra); kinased and hybridized to the complementary synthetic fragment to give

```
5' HO GATCTGTTGGCTCGAGTTGA
           ACAACCGAGCTCAACTAGCT P
       BglII            SalI
```

One pmole of the double-stranded oligonucleotide was then placed in a three way ligation mixture with 1.4 pmoles (0.8 $\mu$g) of Mbo fragment 1 and the vector fragment formed from 1 $\mu$g pBR322 which had been treated with BamHI, SalI and BAP. The mixture was ligated overnight before transforming into E.coli MM294. $Amp^R Tet^S$ colonies were selected and the desired construction confirmed by DNA isolation, restriction analysis and sequencing. The correct plasmid was designated pTS12.

pTS12 (53.5 $\mu$g) was restricted with HhaI blunt-ended with Klenow, 18 $\mu$g of the resulting fragments ligated to 3.15 nmoles of the oligomeric linker CCCCGGGG, and then treated with SmaI. The resulting sequence at the 5' terminus was thus modified to give the sequence GGGGCTGA which encodes the peptide sequence beginning with amino acid 1 of the DT-A fragment, (See Fig 4). The 3' end of the ligation product terminates in the first HhaI site of pBR322 following the SalI site, and the fragment contains the entire coding sequence along with in reading frame with terminator for the small Mbo fragment. The desired 654 bp fragment was isolated using 6% PAGE, and elution by crush and soak.

One picomole of this modified prepared fragment was ligated with 0.7 $\mu$g of pDG141 which had been restricted with SacI, blunt-ended with Klenow, and BAPed (the preparation of pDG141 is described above). The pDG141 derived fragment has an ATG start codon operably linked to the trp promoter. The resulting ligation mixture was transformed into E.coli MM294, and resistant colonies grown in 10 ml TYE' medium containing 100 $\mu$g/ml ampicillin and screened for plasmid size. Those colonies which contained plasmids larger than pDG141 were screened for expression of the DT-A fragment.

The cells were grown to log phase in 10 ml of the TYE'-Amp 100 medium at 37° for 4 hr. To demonstrate expression, one ml of the culture was centrifuged and the pellet resuspended in 20 $\mu$l buffer containing 625 mM Tris, pH 6.8, 3% SDS. After heating at 95°C for 5 min, samples were run in 12.5% SDS-PAGE with a 3% stacking (Laemmli, et al, Nature (1970) 227:680). Two clones which showed an additional protein band at the expected molecule weight were confirmed by the EF-2 ADP-ribosylation assay according to the procedure of Chung, D.W., et al, Infect Immun (1977) 16:832. These colonies, designated ptrpSmlMbo, produced 20 $\mu$g of DT-A per ml of culture. The antigenicity and molecular weight of the product were confirmed by Western Blot.

F.5. Preparation of Expression Vectors $pP_L$MspSA and $pP_L$OPMspSA for Expression of the DT A-B' Toxin-Spacer Construct

One expression vector, $pP_L$MspSA was constructed by inserting the appropriate portions of pATGMspSA behind the $P_L$ promoter. The construction is shown in Fig 3. pATGMspSA was digested with HindIII, PstI, (EcoRI to prevent religation) and the large vector fragment containing the ATG start codon, the A and B' DT toxin and spacer coding sequences used in subsequent ligation. This fragment was then ligated with a PstI, HindIII, BAPed preparation of pFC5 which fragment corresponds to the portion of pFC5 containing the $P_L$ promoter and N-gene ribosome binding site. The ligation mixture was then used to transform MC1000-39351 and transformants selected by $Amp^R$. The correct construction of the desired plasmid $pP_L$MspSA was confirmed by restriction analysis.

A second vector, $pP_L$OPMspSA was constructed by a two-way ligation of a fragment obtained by restricting pCS3 with EcoRI, SalI followed by BAP treatment and a fragment obtained from $pP_L$MspSA by

restriction with EcoRI, SalI and PstI (see Fig 3). The ligation mixture was then used to transform MC1000-39351 and transformants selected by $Amp^R$. The correct construction of the desired plasmid $pP_LOPMspSA$ was confirmed by restriction analysis and Maxam-Gilbert sequencing.

The colonies transformed with each of the foregoing plasmids were grown at 30 °C in TYE medium containing 100 µg/ml ampicillin, and at the end of log phase the temperature raised to 42 °C. After 1.5 hr the cells were centrifuged and sonicated and the sonicate assayed by the assay of Chung, D.W., et al, Infect Immun (1977) 16:832 for enzymatic activity. Activity corresponding to DTA-B'-spacer at levels of 1-10 µg/ml medium was found when $pP_LMspSA$ was used and 20-150 µg/ml when $pP_LOPMspSA$ was used. Production of the desired DT A-B'-spacer was confirmed by Western Blot.

H.1. Assay for Cytotoxicity

The DT-A-B' spacer was conjugated with an antibreast monoclonal antibody 260F9, (hybridoma deposited at the ATCC on 27 January 1984 under accession number HB8488. Ricin toxin A chain (RTA) or diphtheria toxin A chain (DTA) which contain free sulfhydryl groups for analogous conjugation with the antibody were thus conjugated. These conjugates were assayed for immunotoxicity.

To form the conjugate, breast monoclonal antibody 260F9 or other antibodies as specified below were first derivatized with SPDP or

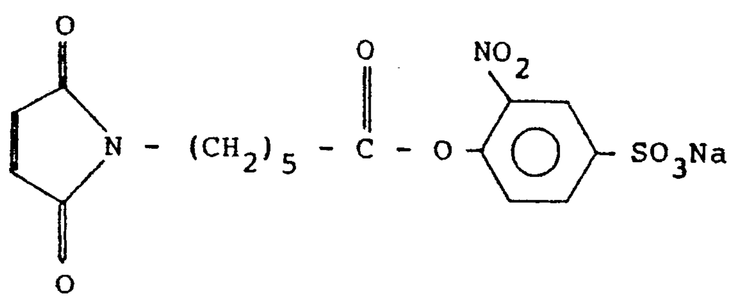

(mal-sac-HNSA). The antibodies derivatized to SPDP were used to form disulfide links to the free cysteine sulfhydryls of DT-A-B'-spacer, DTA or RTA. Those derivatized with mal-sac-HNSA were used to form thioether linkages with DT-A-B'-spacer.

For SPDP, a 10-20 fold molar excess of SPDP was added to a solution containing 20 mg/ml of antibody in PBS and incubated at room temperature for 1 hr, and then dialyzed against PBS to remove unreacted SPDP. It was calculated that approximately 2-5 pyridyl-disulfide moieties were introduced into each antibody using this procedure.

To complete the conjugation with SPDP to give a disulfide linkage to the cytotoxic portions, DT-A-B'-spacer solution or solution of RTA or DTA containing 1-2 mg/ml which had been stored in reducing agent in 4 °C was passed over a Sephadex G-25 column equilibrated in PBS to remove the reducing agent, and the DT-A-B'-spacer or other cytotoxic portion was mixed with derivatized antibody in 2-4 molar excess cytotoxic portion. Conjugation was confirmed by spectrophotometric determination of released pyridine-2-thiol and by SDS-PAGE.

For mal-sac-HNSA, approximately 0.2 ml of mal-sac-HNSA solution containing 1 mg/ml was added to 1 ml antibody solution containing 3-8 mg/ml in PBS. The mixture was kept at room temperature and monitored until 5 mal-sac-HNSA moieties were incorporated per antibody. The reaction was then stopped by desalting the mixture on a G-25 column equilibrated in 0.1 M Na phosphate, pH 6.

The DT-A-B'-spacer, stored in reducing agent at 4 °C, was passed over PBS-equilbiated Sephadex G-25 to remove reducing agent, and the protein (1-2 mg/ml) mixed in 2-4 molar excess with the derivatized antibody. Conjugation was confirmed by SDS-PAGE.

In a typical protocol, breast tumor cells (MCF-7) were seeded in 8 ml glass vials and dilutions of the immunoconjugates were added. Following incubation for 22 hr at 37 °C, the medium was removed and replaced with medium containing $35_S$ methionine. Following a 2-hr pulse, the medium was aspirated, the monolayer was washed twice with 10% trichloroacetic acid containing 1 mg/ml methionine and the vials were dried. Following the addition of 3 ml of 4ad scintillation fluid containing 20% (v/v) Triton X-100, the vials were counted. Toxicity was expressed as the concentration of protein required to inhibit protein synthesis by 50% ($TCID_{50}$).

The results of these assays are shown in Table 1 both for 260F9, and other antibody partners.

Table 1

| Monoclonal Antibody | Ab-DTA | TCID50% (nM) Ab-DT-A-B'-spacer | Ab-RTA |
|---|---|---|---|
| ATR[1] | 10 | 2 | 0.1 |
| 260F9 | 30 | 0.3 | 0.1 |
| 106A10 | > 100 | 7 | 1 |
| 208D11[2] | > 100 | 40 | 4 |
| 245E7[3] | > 100 | 50-100 | 10 |
| MOPC21[4] | > 100 | > 100 | > 100 |

1. positive control, anti-transferrin receptor antibody
2. monoclonal antibreast antibody secreted by hybridoma deposited 1/27/84 at ATCC, number HB8487
3. monoclonal antibreast antibody secreted by hybridoma deposited 1/27/84 at ATCC, number HB8489
4. negative control, purchased from Zymed Labs

The foregoing assay was run as set forth above, using the 260F9 conjugate but substituting alternate cell lines for MCF-7. The results are shown in Table 2.

Table 2

| | TCID50% (nM) | | |
|---|---|---|---|
| | RTA | DT-A-B'-spacer | |
| Cell Line | Disulfide | Disulfide | Thioether |
| MCF-7 | 0.1 | 0.3 | ND |
| CAMA-1 | 0.4 | 1.0 | 0.5 |
| BT-20 | 9 | 1.6 | 1.4 |
| SKBR3 | 0.06 | 0.6 | 0.2 |
| CC95 | > 100 | > 100 | ND |

The cell lines shown, CAMA-1, BT-20, and SKBR-3 are other breast tumor cell lines; a normal fibroblast cell line CC-95 was also used. The DT-A-B'-spacer was comparably active with respect to the alternative breast tumor lines, but relatively inactive against the normal cells.

The materials listed below were deposited with the American Type Culture Collection, Rockville, MD,

USA (ATCC). The deposits were made under the provisions of the Budapest Treaty on the International Recognition of the Deposit of Microorganisms for the Purposes of Patent Procedure and the Regulations thereunder (Budapest Treaty). Maintenance of a viable culture is assured for 30 years from date of deposit. The organism will be made available by ATCC under the terms of the Budapest Treaty, and subject to an agreement between Applicants and ATCC which assures unrestricted availability upon issuance of the pertinent US patent. Availability of the deposited strain is not to be construed as a license to practice the invention in contravention of the rights granted under the authority of any government in accordance with its patent laws.

| Plasmid | Deposit Date | CMCC# | ATCC# |
|---|---|---|---|
| pβ1-Z15 | 13 Jan 1984 | 1948 | 39578 |
| pDG144 | 13 Jan 1984 | -- | 39579 |
| pFC5 | 14 Sept 1984 | 1935 | 39864 |
| pCS3 | 3 June 1982 | -- | 39142 |
| pTRP3 | 18 Dec 1984 | 1731 | 39946 |
| pPLOP | 18 Dec 1984 | 2118 | 39947 |
| pDG141 | 24 Jan 1984 | -- | 39588 |
| pRA123 | 17 Aug 1984 | 2108 | 39799 |
| pRTB704 | 14 Sept 1984 | 1951 | 39865 |
| pRAL6 | 4 Sept 1984 | 2114 | 39833 |
| E. coli K12DG98 | 13 July 1984 | 1965 | 39768 |

## Claims

1. A spacer for separating the cytotoxic portion and the target cell binding portion of a toxin conjugate which spacer comprises an amino acid sequence of the formula:

   $(F\text{-}(Pro)_n)_mF$

   wherein F represents a flexible amino acid sequence wherein each amino acid is individualy selected from the group consisting of serine, glycine, and threonine, n is an integer from 4 to 8 inclusive, and m is an integer from 1 to 4.

2. An amino acid sequence having the formula

   $$\text{Gly-Thr-Gly-Ser-Gly-(Pro)}_6\text{-Ser-Gly-Ser-Gly-Thr-Cys.}$$

3. A DNA sequence which encodes a spacer as claimed in claim 1.

4. A host cell or cell culture transformed with a DNA sequence of claim 3.

5. A fused polypeptide useful in making toxin conjugates comprising:
   (a) a cytotoxic portion; and
   (b) a spacer of claim 1.

6. A polypeptide of claim 5 wherein the cytotoxic portion comprises the enzymatically active fragment of

diphtheria toxin or ricin.

7. A DNA sequence encoding a fused polypeptide of claim 6.

8. A host cell or cell culture transformed with a DNA sequence of claim 7.

9. A conjugated toxin which comprises a fused polypeptide of claim 5 covalently linked to a binding portion, wherein the binding portion is an antibody or a portion thereof.

10. A pharmaceutical or veterinary formulation effective in killing undesirable cells in mammalian subjects comprising a compound of claim 9 formulated for pharmaceutical or veterinary use, respectively, and optionally in admixture with one or more acceptable excipients.

11. The use of a conjugated toxin of claim 9 in the preparation of a pharmaceutical or veterinary formulation for killing undesirable cells.

12. A method for preparing a spacer amino acid sequence as claimed in claim 1 which comprises either;
(a) Synthesising polypeptide subunits by conventional techniques and combining them in sequence by conventional procedures, or
(b) Expressing a DNA sequence encoding for said spacer or subunits thereof, comprising obtaining such a DNA sequence by conventional DNA synthesis techniques or by conventional cloning methods, inserting the DNA sequence into a suitable expression vector, transforming suitable microorganisms or cells with the vector, growing the transformants and harvesting the desired peptides, which peptides, if subunits, may be combined in sequence by conventional procedures.

## Revendications

1. Espaceur pour la séparation de la portion cytotoxique et de la portion se fixant à une cellule cible d'un conjugué de toxine, lequel espaceur comprend une séquence d'amino-acides de formule :

$(F\text{-}(Pro)_n)_mF$

dans laquelle F représente une séquence flexible d'amino-acides dans laquelle chaque amino-acide est individuellement choisi dans le groupe constitué par la sérine, la glycine et la thréonine, n est un entier de 4 à 8 inclusivement et m est un entier de 1 à 4.

2. Séquence d'amino-acides de formule

$$Gly\text{-}Thr\text{-}Gly\text{-}Ser\text{-}Gly\text{-}(Pro)_6\text{-}Ser\text{-}Gly\text{-}Ser\text{-}Gly\text{-}Thr\text{-}Cys.$$

3. Séquence d'ADN qui code pour un espaceur selon la revendication 1.

4. Cellule hôte ou culture de cellules transformée avec une séquence d'ADN de la revendication 3.

5. Polypeptide de fusion utile pour préparer des conjugués de toxines comprenant :
(a) une portion cytotoxique ; et
(b) un espaceur de la revendication 1.

6. Polypeptide selon la revendication 5, dans lequel la portion cytotoxique comprend le fragment enzymatiquement actif de la toxine diphtérique ou de la ricine.

7. Séquence d'ADN codant pour un polypeptide de fusion de la revendication 6.

8. Cellule hôte ou culture de cellules transformée avec une séquence d'ADN de la revendication 7.

9. Toxine conjuguée qui comprend un polypeptide de fusion de la revendication 5 lié par covalence à une

portion fixatrice, où la portion fixatrice est un anticorps ou une portion d'anticorps.

10. Formulation pharmaceutique ou vétérinaire efficace pour tuer des cellules indésirables chez des mammifères, comprenant un composé selon la revendication 9 formulé respectivement pour l'emploi pharmaceutique ou vétérinaire, et facultativement en mélange avec un ou plusieurs excipients acceptables.

11. Utilisation d'une toxine conjuguée de la revendication 9 dans la préparation d'une formulation pharmaceutique ou vétérinaire pour tuer des cellules indésirables.

12. Procédé pour préparer un espaceur constitué d'une séquence d'amino-acides selon la revendication 1 qui comprend soit :
    (a) la synthèse de sous-unités polypeptidiques selon des techniques classiques et leur combinaison en une séquence selon des modes opératoires classiques, ou
    (b) l'expression d'une séquence d'ADN codant pour ledit espaceur ou des sous-unités de celui-ci, comprenant l'obtention d'une telle séquence d'ADN selon des techniques classiques de synthèse d'ADN ou selon des procédés classiques de clonage, l'insertion de la séquence d'ADN dans un vecteur d'expression approprié, la transformation de microorganismes ou de cellules appropriés avec le vecteur, la culture des transformants et la récolte des peptides désirés, lesquels peptides, lorsqu'ils constituent des sous-unités, peuvent être combinés en une séquence selon des modes opératoires classiques.

**Patentansprüche**

1. Brückensequenz zur Trennung des cytotoxischen Teils und des Zielzellen-bindenden Teils eines Toxin-Konjugats, umfassend eine Aminosäuresequenz der Formel:

   $(F-(Pro)_n)_mF$

   in der F eine flexible Aminosäuresequenz darstellt, wobei jede Aminosäure einzeln aus der Gruppe Serin, Glycin und Threonin ausgewählt ist, n einen Wert von 4 bis einschließlich 8 und m eine ganze Zahl mit dem Wert von 1 bis 4 ist.

2. Aminosäuresequenz der Formel

   $$\text{Gly-Thr-Gly-Ser-Gly-(Pro)}_6\text{-Ser-Gly-Ser-Gly-Thr-Cys.}$$

3. DNA-Sequenz, die für eine Brückensequenz nach Anspruch 1 codiert.

4. Wirtszelle oder Zellkultur, die mit einer DNA-Sequenz nach Anspruch 3 transformiert ist.

5. Fusioniertes Polypeptid, das zur Herstellung von toxischen Konjugaten verwendbar ist, umfassend:
    (a) einen cytotoxischen Teil; und
    (b) eine Brückensequenz nach Anspruch 1.

6. Polypeptid nach Anspruch 5, bei dem der cytotoxische Teil das enzymatisch aktive Fragment von Diphtherietoxin oder Ricin umfaßt.

7. DNA-Sequenz, die für ein fusioniertes Polypeptid nach Anspruch 6 codiert.

8. Wirtszelle oder Zellkultur, die mit einer DNA-Sequenz nach Anspruch 7 transformiert ist.

9. Konjugiertes Toxin, umfassend ein fusioniertes Polypeptid nach Anspruch 5, das kovalent an einen bindenden Teil gebunden ist, wobei der bindende Teil ein Antikörper oder ein Teil davon ist.

10. Pharmazeutische oder veterinäre, für das Abtöten ungewünschter Zellen in Säugern wirksame Formulierung umfassend eine Verbindung nach Anspruch 9, die für eine pharmazeutische bzw. veterinäre Verwendung formuliert ist, gegebenenfalls im Gemisch mit einem oder mehreren geeigneten Arzneimittelträgern.

11. Verwendung eines konjugierten Toxins nach Anspruch 9 zur Herstellung einer pharmazeutischen oder veterinären Formulierung für das Abtöten ungewünschter Zellen.

12. Verfahren zur Herstellung einer Aminosäurebrückensequenz nach Anspruch 1, umfassend entweder
(a) die Synthese von Polypeptid-Untereinheiten durch übliche Techniken und deren Zusammenfügen zu einer Sequenz durch übliche Verfahren, oder
(b) die Expression einer für die Brückensequenz oder Untereinheiten davon codierenden DNA-Sequenz, umfassend den Erhalt einer solchen DNA-Sequenz durch übliche DNA-Synthesemethoden oder durch übliche Clonierungsmethoden, das Einfügen der DNA-Sequenz in einen geeigneten Expressionsvektor, die Transformation geeigneter Mikroorganismen oder Zellen mit dem Vektor, die Züchtung der Transformanten und die Gewinnung der gewünschten Peptide, die, wenn sie aus Untereinheiten bestehen, durch übliche Verfahren zu einer Sequenz zusammengefügt werden können.

Hap I / Mbo I

CCGGCGTTGCG TATCCAGTGGCTACACTCAGGTTGTAATGA TTGGGATGATGTACCTGATCTGAGAGCGAT

TAAAAACTCATTGAGGAGTAGGTCCCGATT GGTTTTTGCTAGTGAAGCTTAGCTAGCTTT CCCCATGTAACCAATCTATCAAAAAAGGGC ATTGATTTCAGAGCACCCTTATAATTAGGA

TAGCTTTACCTAATTATTTTATGAGTCCTG GTAAGGGGATACGTTGTGAGCAGAAAACTG TTTGCGTCAATCTTAATAGGGGCGCTACTG GGGATAGGGGCCCCACCTTCAGCCCATGCA
TyrValLeu ValArgGlyTyrValValSerArgLysLeu PheAlaSerIleLeuIleGlyAlaLeuLeu GlyIleGlyAlaProProSerAlaHisAla
-31 -21 -11 -1

GGCGCTGATGATGTTGTTGATTCTTCTAAA TCTTTTGTGATGGAAAACTTTTCTTCGTAC CACGGGACTAAACCTGGTTATGTAGATTCC ATTCAAAAAGGTATACAAAAGCCAAAATCT
GlyAlaAspAspValValAspSerSerLys SerPheValMetGluAsnPheSerSerTyr HisGlyThrLysProGlyTyrValAspSer IleGlnLysGlyIleGlnLysProLysSer
10 20 30 40

GGTACACAAGGAAATTATGACGATGATTGG AAAGGGTTTTATAGTACCGACAATAAATAC GACGCTGCGGGATACTCTGTAGATAATGAA AACCCGCTCTCTGGAAAAGCTGGAGGCGTG
GlyThrGlnGlyAsnTyrAspAspAspTrp LysGlyPheTyrSerThrAspAsnLysTyr AspAlaAlaGlyTyrSerValAspAsnGlu AsnProLeuSerGlyLysAlaGlyGlyVal
50 60 70 80

GTCAAAGTGACGTATCCAGGACTGACGAAG GTTCTCGCACTAAAAGTGGATAATGCCGAA ACTATTAAGAAAGAGTTAGGTTTAAGTCTC ACTGAACCGTTGATGGAGCAAGTCGGAACG
ValLysValThrTyrProGlyLeuThrLys ValLeuAlaLeuLysValAspAsnAlaGlu ThrIleLysLysGluLeuGlyLeuSerLeu ThrGluProLeuMetGluGlnValGlyThr
90 100 110 120

GAAGAGTTTATCAAAAGGTTCGGTGATGGT GCTTCGCGTGTAGTGCTCAGCCTTCCCTTC GCTGAGGGGAGTTCTAGCGTTGAATATATT AATAACTGGGAACAGGCGAAAGCGTTAAGC
GluGluPheIleLysArgPheGlyAspGly AlaSerArgValValLeuSerLeuProPhe AlaGluGlySerSerSerValGluTyrIle AsnAsnTrpGluGlnAlaLysAlaLeuSer
130 140 150 160

Mbo I

GTAGAACTTGAGATTAATTTTGAAACCCGT GGAAAACGTGGCCAAGATGCGATGTATGAG TATATGGCTCAAGCCTGTGCAGGAAATCGT GTCAGGCGATCAGTAGGTAGCTCATTGTCA
ValGluLeuGluIleAsnPheGluThrArg GlyLysArgGlyGlnAspAlaMetTyrGlu TyrMetAlaGlnAlaCysAlaGlyAsnArg ValArgArgSerValGlySerSerLeuSer
170 180 190 200

TGCATAAATCTTGATTGGGATGTCATAAGG GATAAAACTAAGACAAAGATAGAGTCTTTG AAAGAGCATGGCCCTATCAAAAATAAAATG AGCGAAAGTCCCAATAAAACAGTATCTGAG
CysIleAsnLeuAspTrpAspValIleArg AspLysThrLysThrLysIleGluSerLeu LysGluHisGlyProIleLysAsnLysMet SerGluSerProAsnLysThrValSerGlu
210 220 230 240

DIPHTHERIA TOXIN CODING & AMINO ACID SEQUENCE

FIG. 1-1

```
GAAAAAGCTAAACAATACCTAGAAGAATTT CATCAAACGGCATTAGAGCATCCTGAATTG TCAGAACTTAAAACCGTTACTGGGACCAAT CCTGTATTCGCTGGGGCTAACTATGCGGCG
GluLysAlaLysGlnTyrLeuGluGluPhe HisGlnThrAlaLeuGluHisProGluLeu SerGluLeuLysThrValThrGlyThrAsn ProValPheAlaGlyAlaAsnTyrAlaAla
                           250                            260                            270                            280

TGGGCAGTAAACGTTGCGCAAGTTATCGAT AGCGAAACAGCTGATAATTTGGAAAAGACA ACTGCTGCTCTTTCGATACTTCCTGGTATC GGTAGCGTAATGGGCATTGCAGACGGTGCC
TrpAlaValAsnValAlaGlnValIleAsp SerGluThrAlaAspAsnLeuGluLysThr ThrAlaAlaLeuSerIleLeuProGlyIle GlySerValMetGlyIleAlaAspGlyAla
                           290                            300                            310                            320

GTTCACCACAATACAGAAGAGATAGTGGCA CAATCAATAGCTTTATCGTCTTTAATGGTT GCTCAAGCTATTCCATTGGTAGGAGAGCTA GTTGATATTGGTTTCGCTGCATATAATTTT
ValHisHisAsnThrGluGluIleValAla GlnSerIleAlaLeuSerSerLeuMetVal AlaGlnAlaIleProLeuValGlyGluLeu ValAspIleGlyPheAlaAlaTyrAsnPhe
                           330                            340                            350                            360
                                                                   Msp I
GTAGAGAGTATTATCAATTTATTTCAAGTA GTTCATAATTCGTATAATCGTCCCGCGTAT TCTCCGGGGCATAAAACGCAACCATTTCTT CATGACGGGTATGCTGTCAGTTGGAACACT
ValGluSerIleIleAsnLeuPheGlnVal ValHisAsnSerTyrAsnArgProAlaTyr SerProGlyHisLysThrGlnProPheLeu HisAspGlyTyrAlaValSerTrpAsnThr
                           370                            380                            390                            400

GTTGAAGATTCGATAATCCGAACTGGTTTT CAAGGGGAGAGTGGGCACGACATAAAAATT ACTGCTGAAAATACCCCGCTTCCAATCGCG GGTGTCCTACTACCGACTATTCCTGGAAAG
ValGluAspSerIleIleArgThrGlyPhe GlnGlyGluSerGlyHisAspIleLysIle ThrAlaGluAsnThrProLeuProIleAla GlyValLeuLeuProThrIleProGlyLys
                           410                            420                            430                            440

CTGGACGTTAATAAGTCCAAGACTCATATT TCCGTAAATGGTCGGAAAATAAGGATGCGT TGCAGAGCTATAGACGGTGATGTAACTTTT TGTCGCCCTAAATCTCCTGTTTATGTTGGT
LeuAspValAsnLysSerLysThrHisIle SerValAsnGlyArgLysIleArgMetArg CysArgAlaIleAspGlyAspValThrPhe CysArgProLysSerProValTyrValGly
                           450                            460                            470                            480
                                                                                                                   Mbo I
AATGGTGTGCATGCGAATCTTCACGTGGCA TTTCACAGAAGCAGCTCGGAGAAAATTCAT TCTAATGAAATTTCGTCGGATTCCATAGGC GTTCTTGGGTACCAGAAAACAGTAGATCAC
AsnGlyValHisAlaAsnLeuHisValAla PheHisArgSerSerSerGluLysIleHis SerAsnGluIleSerSerAspSerIleGly ValLeuGlyTyrGlnLysThrValAspHis
                           490                            500                            510                            520
                                                                        Msp I
ACCAAGGTTAATTCTAAGCTATCGCTATTT TTTGAAATCAAAAGCTGAAAGGTAGTGGGG TCGTGTGCCGG
ThrLysValAsnSerLysLeuSerLeuPhe PheGluIleLysSerTer
                           530
```

DIPHTHERIA TOXIN CODING & AMINO ACID SEQUENCE

FIG. 1-2

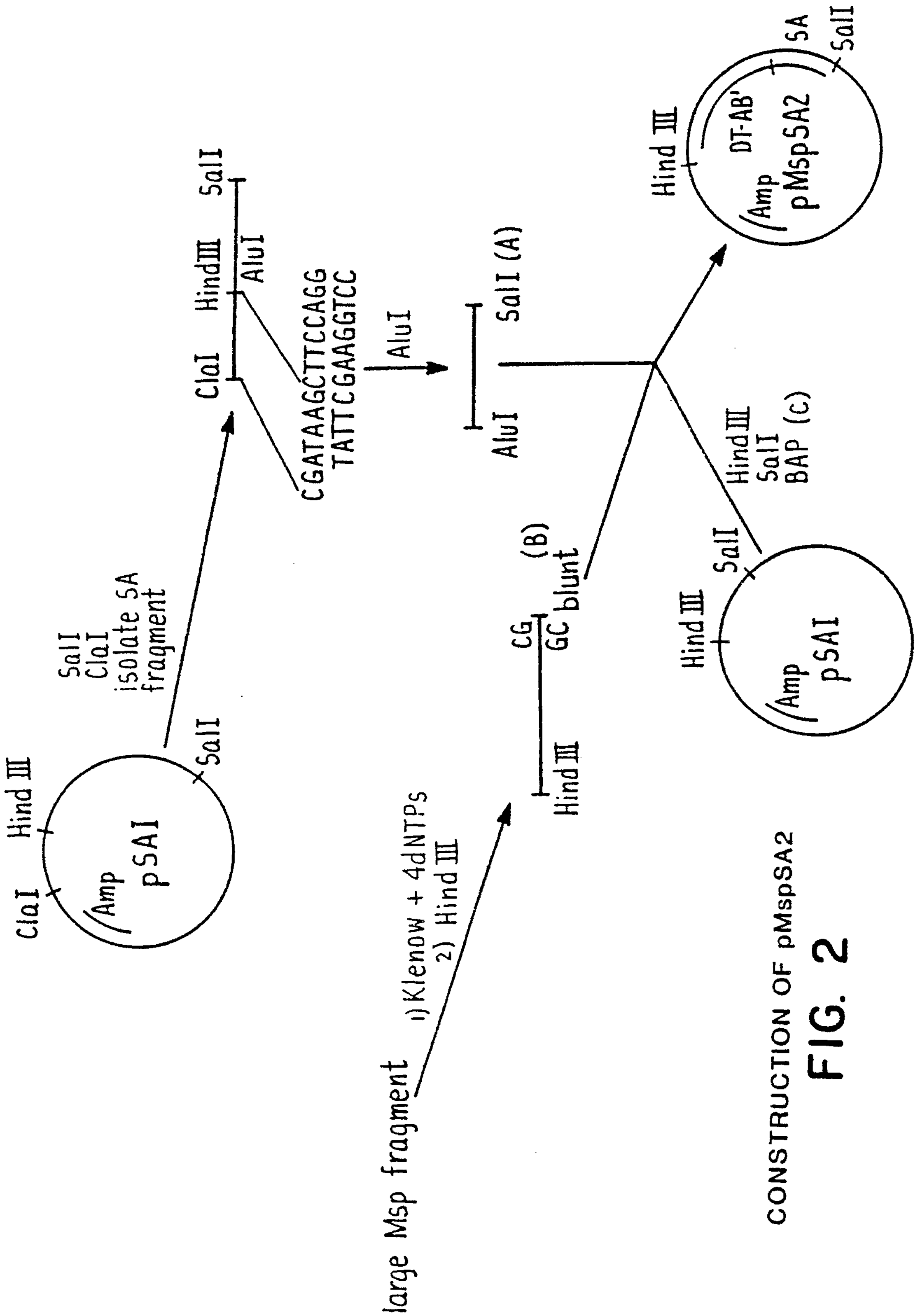


CONSTRUCTION OF pMspSA2

FIG. 2

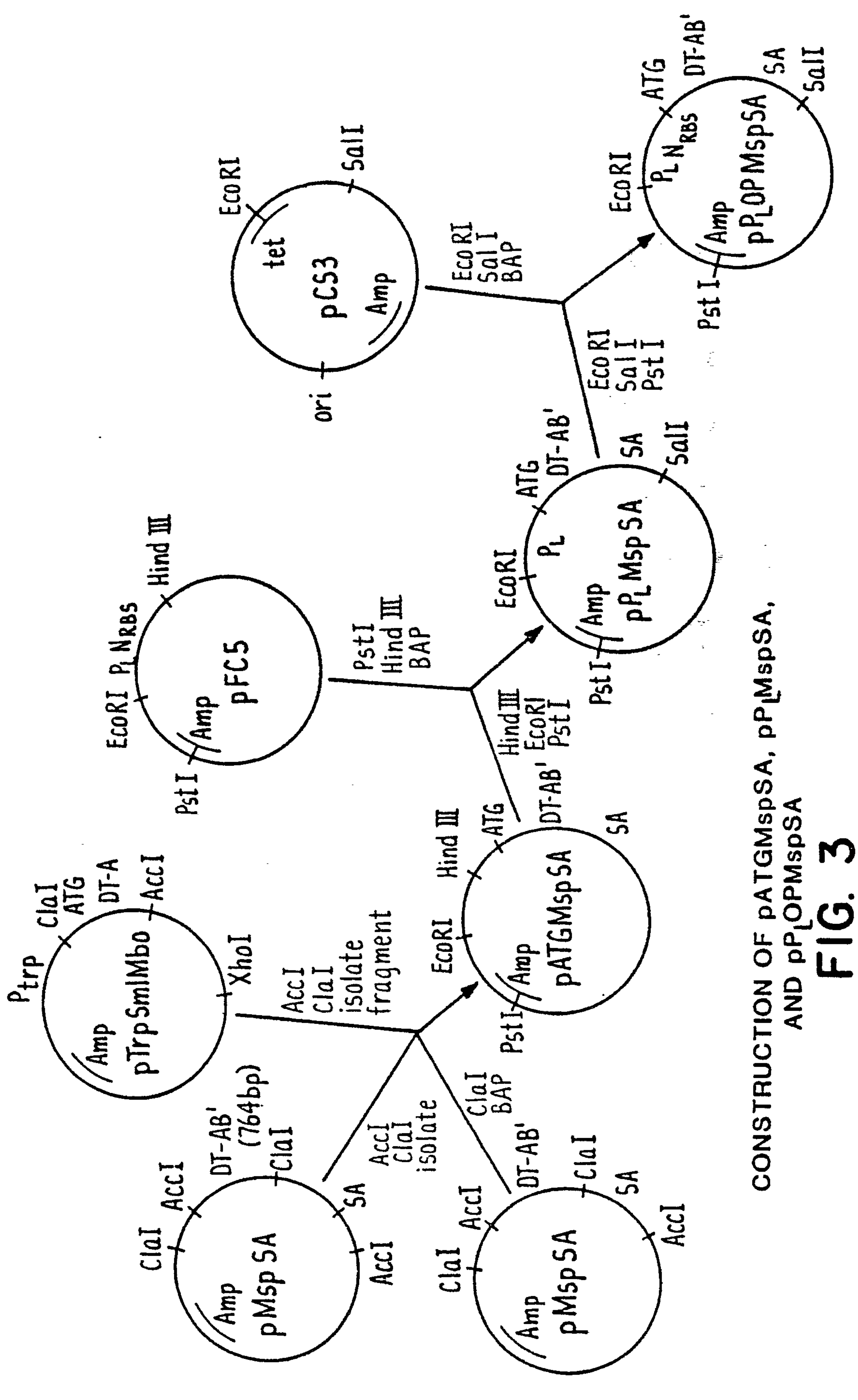


CONSTRUCTION OF pATGMspSA, pP$_L$MspSA, AND pP$_L$OPMspSA

FIG. 3

FIG. 4

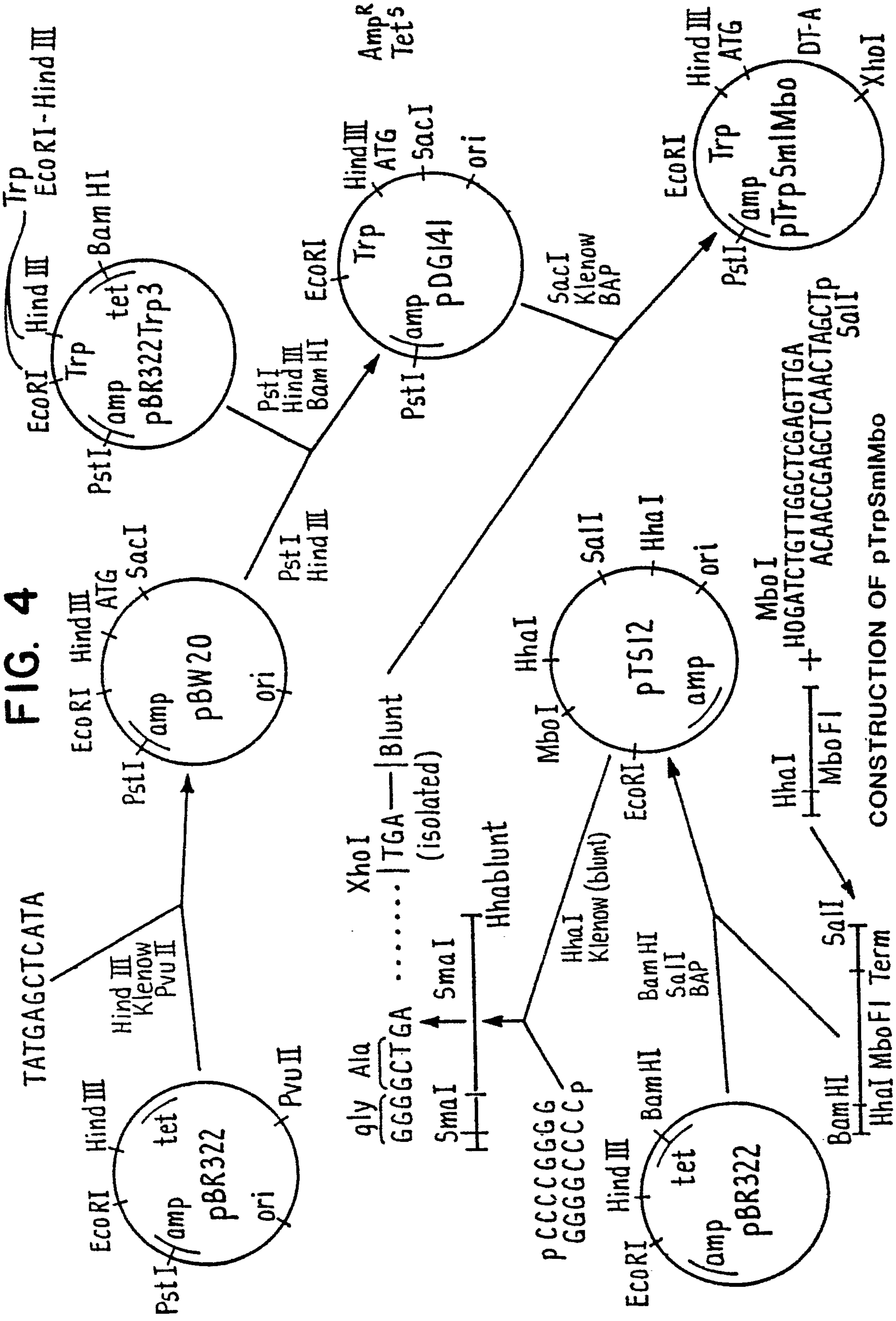


CONSTRUCTION OF pTrpSmlMbo

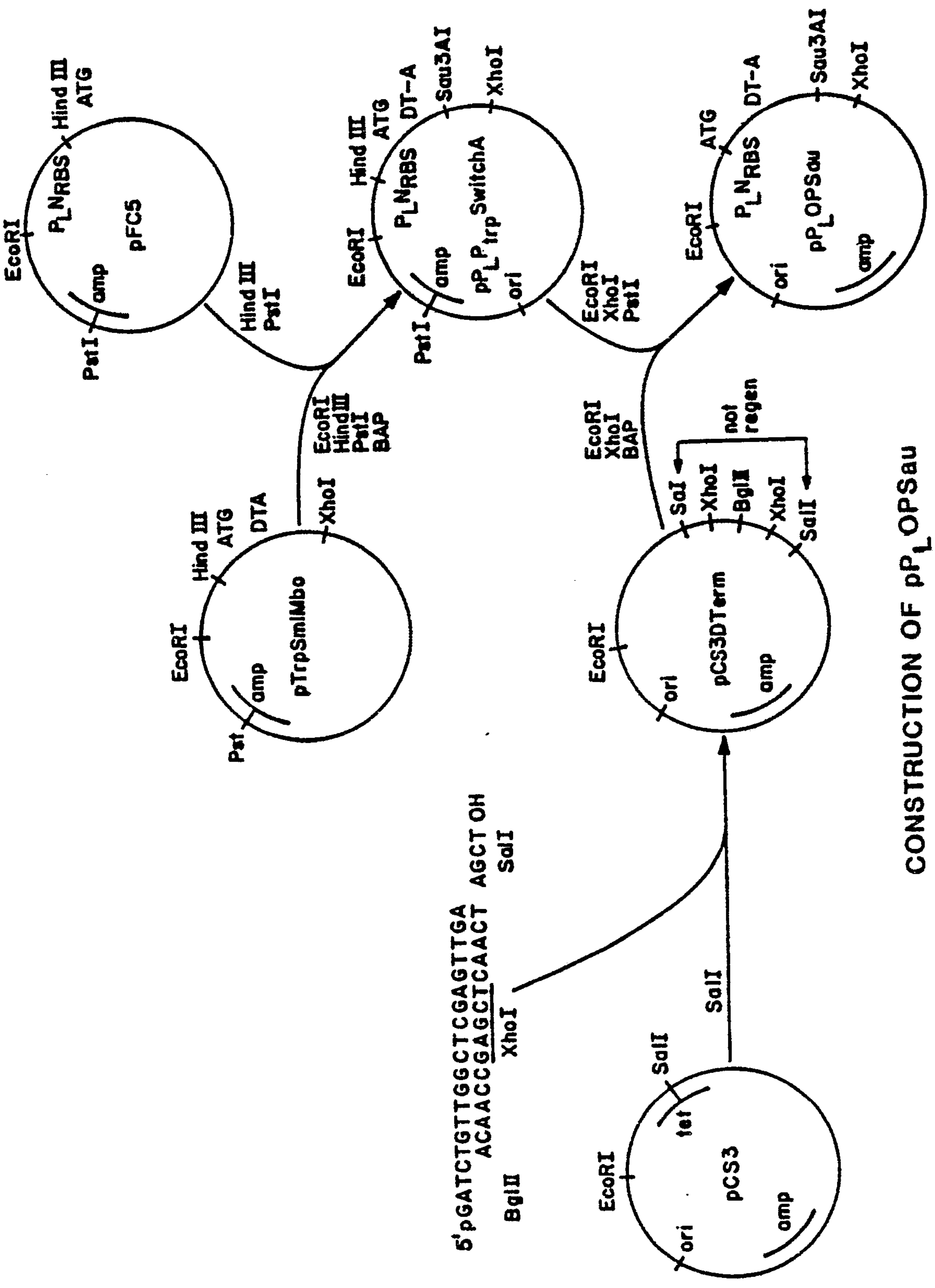


CONSTRUCTION OF $pP_L$OPSau

FIG. 5

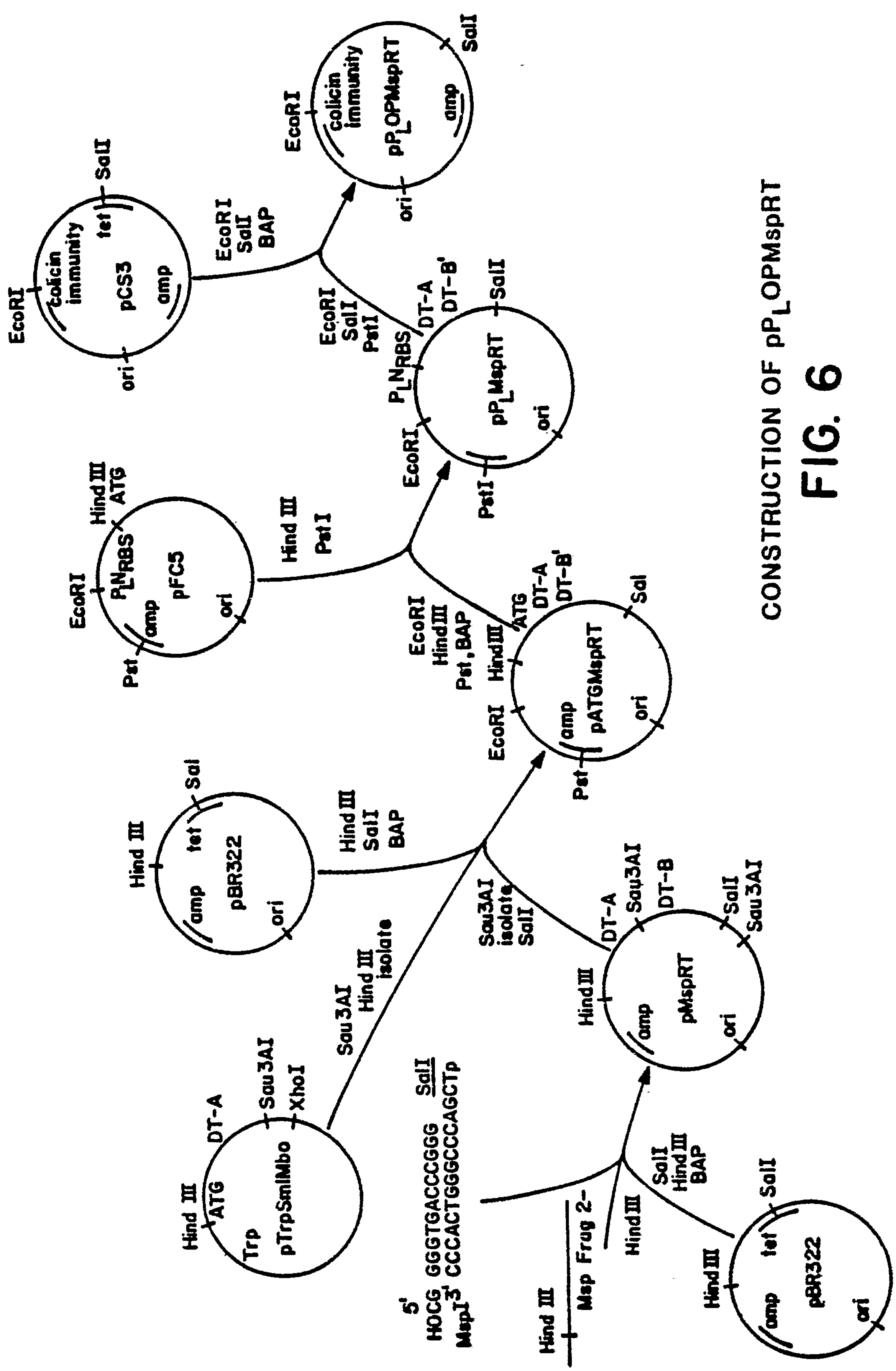


CONSTRUCTION OF $pP_LOPMspRT$

FIG. 6

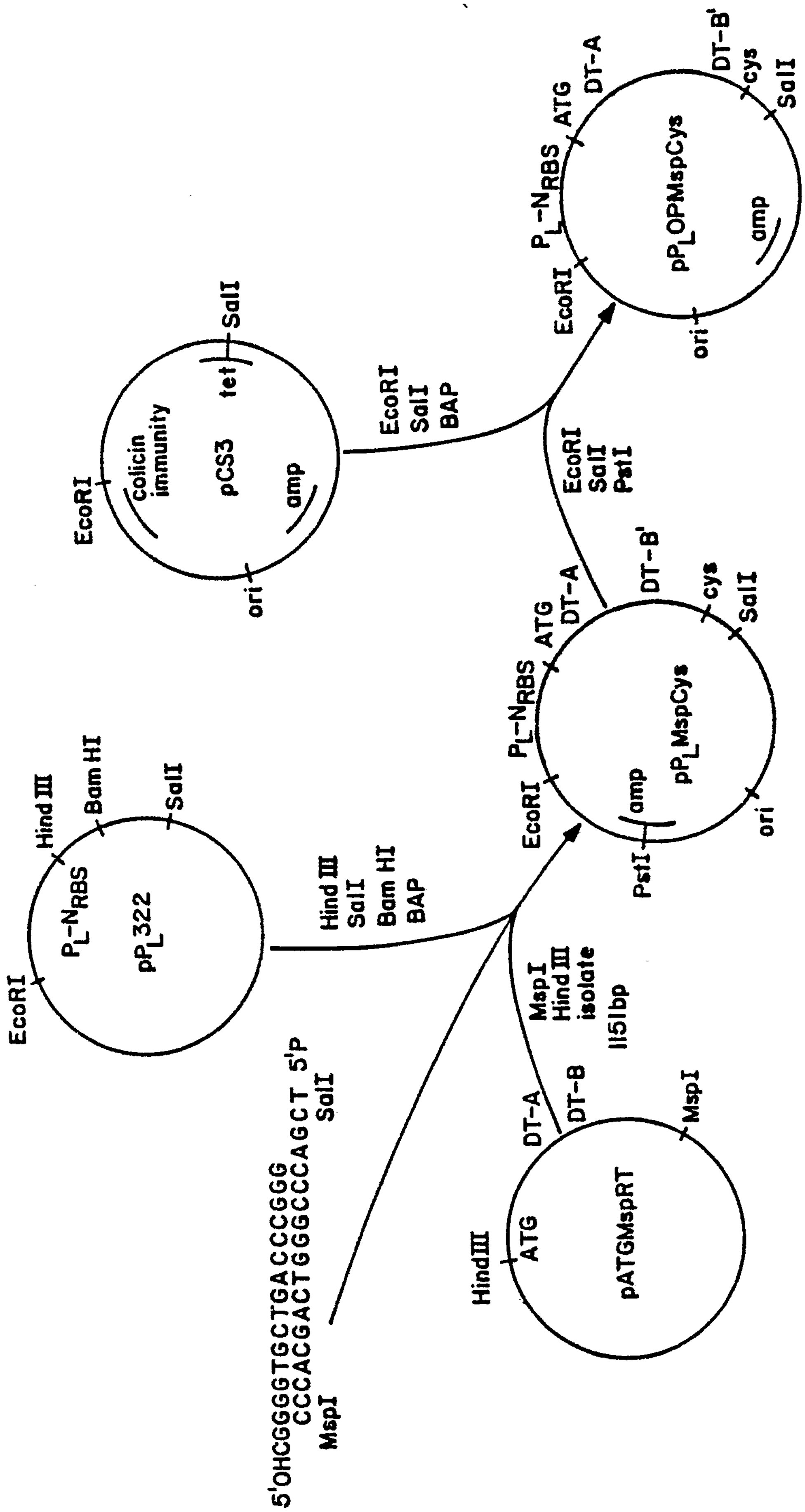


CONSTRUCTION OF $pP_L$OPMspCys

FIG. 7

# FIG. 8

Results of Polyacrylamide Gel Electrophoresis of Extracts of Transformed Cells

Lanes 2-9 represent extracts of MC1000-39531 cultures transformed with the plasmids of the invention as follows:

Lanes 2 and 3: $p^{P}_{L}$OPSau

Lanes 4 and 5: $p^{P}_{L}$OPMspSA

Lanes 6 and 7: $p^{P}_{L}$OPMspRT

Lanes 8 and 9: $p^{P}_{L}$OPMspCys

Lanes 2, 4, 6 and 8 are extracts of cells which were induced by an increase in medium temperature to 42° as described, Lanes 3, 5, 7 and 9 are extracts from uninduced cells.

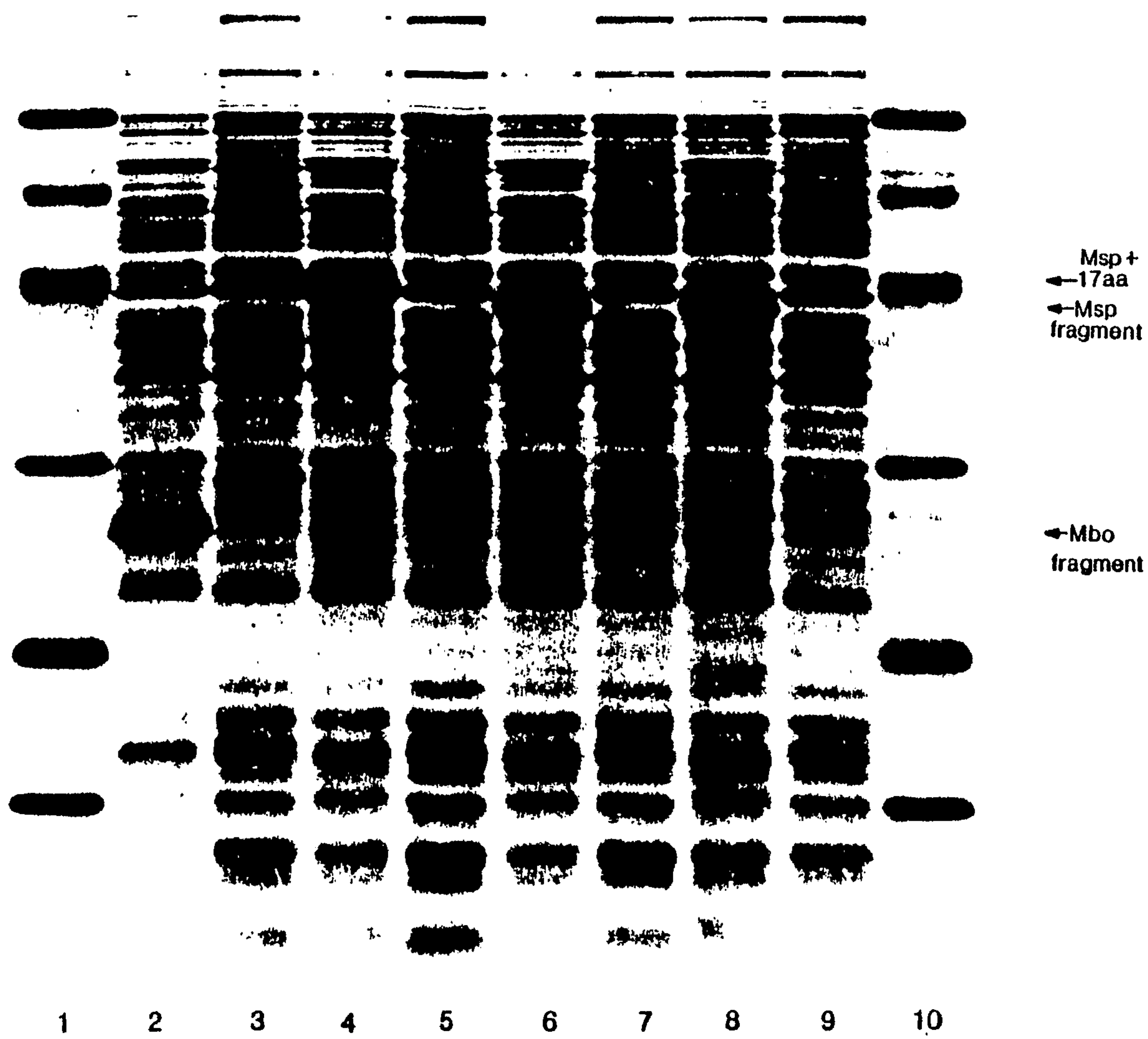

Sequences of pRTA-115, pRTB-4 and pRTB-5 Cloned Inserts

115-TATTAATCCCTATCATAGCTCTCATGGTGTATAGATGCGCACCTCCACCGTCGTCACAGTT

115-TTCTTTGCTTATAAGGCCAGTGGTGCCAAATTTTAATGCTGATGTTTGTATGGATCCTGAGC

115 --(EcoRI)-- 4

115-CCATAGTGCGTATCGTAGGTCGAAATGGTCTATGTGTTGATGTTACAGGTGAAGAATTCTAC
5-gaattccGCGTATCGTAGGTCGAAATGGTCTATGTGTTGATGTTAGGGATGGAAGATTCCAC

4-GATGGAAACCCAATACAATTGTGGCCTTGCAAATCTAATACAGACTGGAATCAGTTATGGA
5-AACGGAAACGCAATACAGTTGTGGCCATGCAAGTCTAATACAGATGCAAATCAGCTCTGGA

4-CTTTGAGAAAAGACGGTACAATTCGATCTAATGGCAAGTGTTTGACCATTTATAAGTCCAG
5-CTTTGAAAAGAGACAATACTATTCGATCTAATGGAAAGTGTTTAACTACTTACGGGTACAG

4-TCTAGGAAAGCATGTGATGATATATAATTGTACTACCGCTACAGTTGGTGCCACCCGTTGG
5-TCCGGGAGTCTATGTGATGATCTATGATTGCAATACTGCTGCAACTGATGCCACCCGCTGG

4-CAAATATGGGACAACCGAACCATCATAAATCCCATATCTGGTTTAGTTTTGGCAGCCACAT
5-CAAATATGGGATAATGGAACCATCATAAATCCCAGATCTAGTCTAGTTTTAGCAGCGACAT

4-CAGGAAACAGTGGTACCACACTTACAGTGCAAACCAACATTTATGCCGTTAGTCAAGGTTG
5-CAGGCAACAGTGGTACCACACTTACAGTGCAAACCAACATTTATGCCGTTAGTCAAGGTTG

4-GCTTCCTAGTAATAATACACAACCTTTTGTGACATCCATTGTTGGGCTAAATGATCTCTGT
5-GCTTCCTACTAATAATACACAACCTTTTGTGACAACCATTGTTGGGCTATACGGTCTGTGC

4-TTACAAGCAAATACTGGAAAAGTATGGTTAGACGAGTGTACAAGTGAAAAGGCTGAACAAC
5-TTGCAAGCAAATAGTGGACAAGTATGGATAGAGGACTGTAGCAGTGAAAAGGCTGAACAAC

4-AATGGGCGCTTTATGCAGATGGTTCAATACGGCCTCAGCAAAACCAAGATAACTGCCTTAC
5-AGTGGGCTCTTTATGCAGATGGTTCAATACGTCCTCAGCAAAACCGAGATAATTGCCTTAC

4-AAGTGATGCTAATATACGAGAAACAATTGTCAAGACCCTCTCTTGCAGCACTGCATCCTCC
5-AAGTGATTCTAATATACGGGAAACAGTTGTCAAGATCCTCTCTTGTGGCCCTGCATCCTCT

4-GGCCAGCGATGGATGTTCAAGAATGATGGAACCATTTGGAATTTGTATAATGGATTGGTGT
5-GGCCAACGATGGATGTTCAAGAATGATGGAACCATTTTAAATTTGTATAGTGGGTTGGTGT

4-TAGATGTGAAGCGATCGGATCCGACCCTTAAACAAATCATTATTTACCCTTTCCATGGAAA
5-TAGATGTGAGGGCATCGGATCCGAGCCTTAAACAAATCATTCTTTACCCTCTCCATGGTGA

4-CCCAAACCAAATATGGTTTCCACTATTTTGATAGACTAATTACCCTCTTGCAGTGTATGTA
5-CCCAAACCAAATATGGTTACCATTATTTTGATAGACAGATTACTCTCTTGCAGTGTGTATG

4-TGTCCTACCATGAACATAGTTG CTTAAATAAAAAGGACATTGTAAATTAAAAAAA...
5-  TCCTGCCATGAAAATAGATGGCTTAAATAAAAAGGACATTGTAAATTTTGTAACTGAAA

5-GGACAGCAAGTTATTGCAGTCCAGTATCTAATAAGAGCACAACTATTGTCTTGTGCAAAAAA...

FIG. 9

# FIG. 10

Comparison of Ricin-B Sequence with RTB-5

```
R-AlaAspValCysMetAspProGluProIleValArgIleValGlyArgAsnGlyLeuCys
                                      ArgIleValGlyArgAsnGlyLeuCys
                               gaattccGCGTATCGTAGGTCGAAATGGTCTATGT

R-ValAsnValArgAspGlyArgPheAsnHisGlyAsnAlaIleGlnLeuTrpProCysLys
  ValAspValArgAspGlyArgPheHisAsnGlyAsnAlaIleGlnLeuTrpProCysLys
  GTTGATGTTAGGGATGGAAGATTCCACAACGGAAACGCAATACAGTTGTGGCCATGCAAG

R-SerAsnThrAspAlaAsnGlnLeu   ThrLeuLysArgAspAsnThrIleArgSerAsn
  SerAsnThrAspAlaAsnGlnLeuTrpThrLeuLysArgAspAsnThrIleArgSerAsn
  TCTAATACAGATGCAAATCAGCTCTGGACTTTGAAAAGAGACAATACTATTCGATCTAAT

R-GlyLysCysLeuThrThrTyrGlyTyrProSerGlyValTyrValMetIleTyrAspCys
  GlyLysCysLeuThrThrTyrGlyTyrSerProGlyValTyrValMetIleTyrAspCys
  GGAAAGTGTTTAACTACTTACGGGTACAGTCCGGGAGTCTATGTGATGATCTATGATTGC

R-AsnThrAlaAlaThrThrAlaAspArg   GluIleTrpAsnAsnGlyThrIleIleAsnPro
  AsnThrAlaAlaThrAspAlaThrArgTrpGlnIleTrpAspAsnGlyThrIleIleAsnPro
  AATACTGCTGCAACTGATGCCACCCGCTGGCAAATATGGGATAATGGAACCATCATAAATCCC

R-ArgSerSerLeuValLeuAlaAlaThrSerGlyAsnSerGlyThrThrLeuThrValGlnThr
  ArgSerGlyLeuValLeuAlaAlaThrSerGlyAsnSerGlyThrThrLeuThrValGlnThr
  AGATCTAGTCTAGTTTTAGCAGCGACATCAGGCAACAGTGGTACCACACTTACAGTGCAAACC

R-AsnIleTyrAlaValSerGlnGlyProLeuPheThrAsnAsnThrGlnProTrpValThr
  AsnIleTyrAlaValSerGlnGlyTrpLeuProThrAsnAsnThrGlnProPheValThr
  AACATTTATGCCGTTAGTCAAGGTTGGCTTCCTACTAATAATACACAACCTTTTGTGACA

R-ThrIleValGlyLeuTyrGlyLeuCysLeuGlnAlaAsnSerGlyGlnValValIleGlu
  ThrIleValGlyLeuTyrGlyLeuCysLeuGlnAlaAsnSerGlyGlnValTrpIleGlu
  ACCATTGTTGGGCTATACGGTCTGTGCTTGCAAGCAAATAGTGGACAAGTATGGATAGAG

R-AspSerCysSerGluLysAlaGluGlnGlnTrpAlaLeuTyrAlaSerGlyAsnIleAsn
  AspCysSerSerGluLysAlaGluGlnGlnTrpAlaLeuTyrAlaAspGlySerIleArg
  GACTGTAGCAGTGAAAAGGCTGAACAACAGTGGGCTCTTTATGCAGATGGTTCAATACGT

R-ProGlnGlnArgArgAspAsnCysLeuThrSerAspSerAsnIleArgGluThrValVal
  ProGlnGlnAsnArgAspAsnCysLeuThrSerAspSerAsnIleArgGluThrValVal
  CCTCAGCAAAACCGAGATAATTGCCTTACAAGTGATTCTAATATACGGGAAACAGTTGTC

R-LysIleLeuSerCysGlyProAlaSerSerGlyGluArgTrpMetPheLysAsnAspGly
  LysIleLeuSerCysGlyProAlaSerSerGlyGlnArgTrpMetPheLysAsnAspGly
  AAGATCCTCTCTTGTGGCCCTGCATCCTCTGGCCAACGATGGATGTTCAAGAATGATGGA

R-ThrIleLeuAsnLeuTyrSerGlyLeuValLeuAspValArgAlaSerAspProSerLeu
  ThrIleLeuAsnLeuTyrSerGlyLeuValLeuAspValArgAlaSerAspProSerLeu
  ACCATTTTAAATTTGTATAGTGGGTTGGTGTTAGATGTGAGGGCATCGGATCCGAGCCTT

R-LysGlnIleIleLeuTyrProLeuTrpGlyHisAspProAsnGlnLeu   IleLeuProPhe
  LysGlnIleIleLeuTyrProLeu   HisGlyAspProAsnGlnIleTrpLeuProLeuPheTerTer
  AAACAAATCATTCTTTACCCTCTC   CATGGTGACCCAAACCAAATATGGTTACCATTATTTTGATAG

  ACAGATTACTCTCTTGCAGTGTGTATGTCCTGCCATGAAAATAGATGGCTTAAATAAAAA
  GGACATTGTAAATTTTGTAACTGAAAGGACAGCAAGTTATTGCAGTCCAGTATCTAATAA
  GAGCACAACTATTGTCTTGTGCAAAAAA....
```

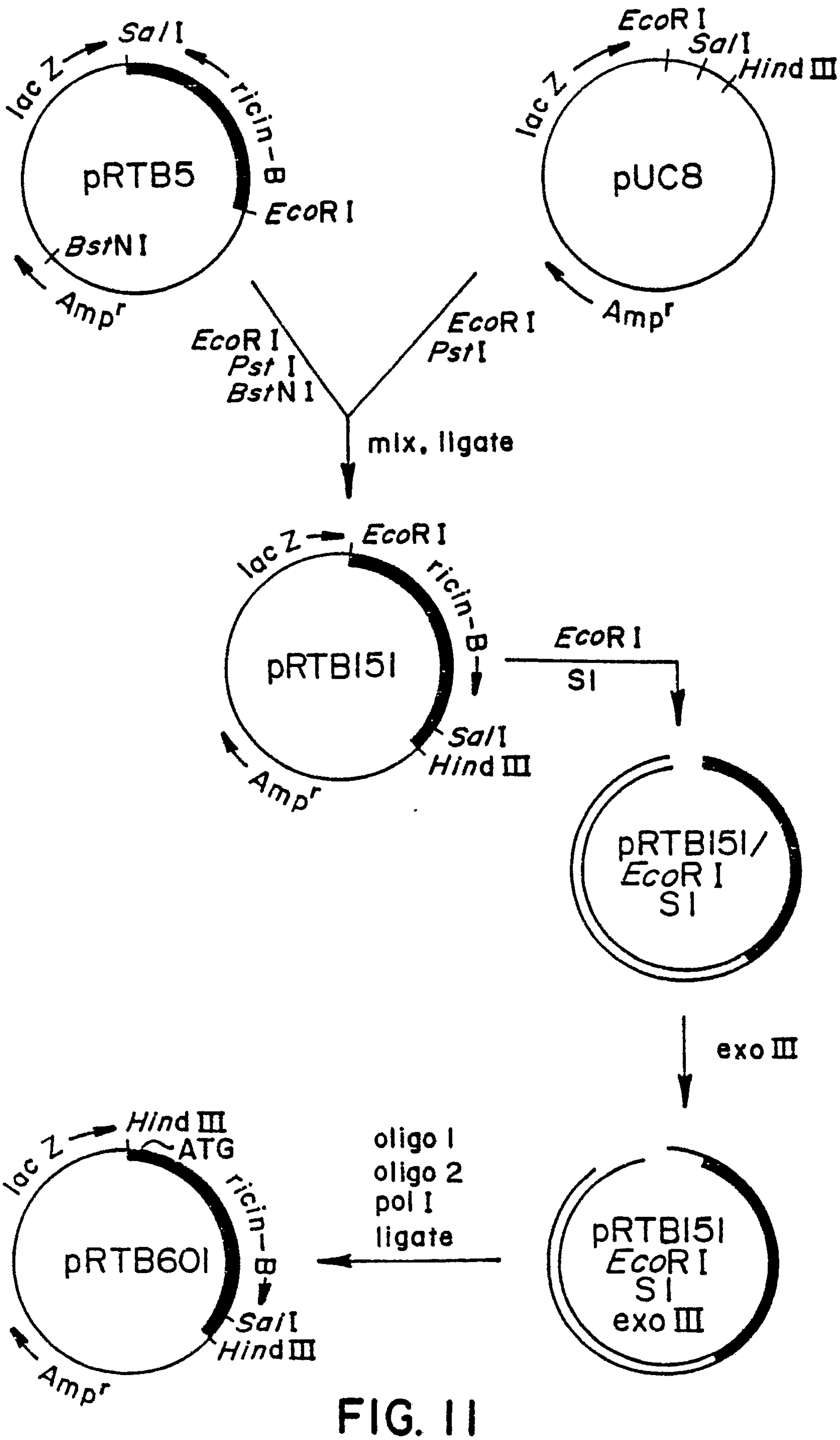
Sal I
lac Z
ricin-B
pRTB5
EcoR I
BstN I
Ampr
EcoR I
Sal I
Hind III
lac Z
pUC8
Ampr
EcoR I
Pst I
BstN I
EcoR I
Pst I
mix, ligate
lac Z
EcoR I
ricin-B
pRTB151
Sal I
Hind III
Ampr
EcoR I
S1
pRTB151/
EcoR I
S1
exo III
Hind III
ATG
lac Z
ricin-B
pRTB601
Sal I
Hind III
Ampr
oligo 1
oligo 2
pol I
ligate
pRTB151
EcoR I
S1
exo III

FIG. 11

## FIG. 12a

Junction Region for the Construction of pRTB601

```
                                                    |------  pRTB-151  --->
        Hind III |--------------  ricin-B  ---------------------------->
               MetAlaAspValCysMetAspProGluProIleValArgIleValGlyArgAsnGlyLeu...
GACCATGATAAGCTTATGGCTGATGTTTGTATGGATCC            GCGTATCGTAGGTCGAAATGGTCTA...
                             TACCTAGGACTCGGGTATCACGCATAGCATCC      ACCAGAT...
        (oligo 2)                        (oligo 1)
```

## FIG. 12b

Fusion of lacZ and Ricin-B in pRTB236

```
   <------ lacZ -----------|  |----------- ricin-B ---------------->
               MetThrMetIleThrProArgIleValGlyArgAsnGlyLeuCysValAspValArgAsp...
...CAGGAAACAGCTATGACCATGATTACGCCGCGTATCGTAGGTCGAAATGGTCTATGTGTTGATGTTAGGGAT...
   (RBS)
```

## FIG. 12c

Junction Region in pRTB514

```
                                  |----------- ricin-B ------------->
  <------ pDG141 --------||-------------- pRTB601 ---------------->
                                 MetAlaAspValCysMetAspProGluProIleValArgIle...
...AGTTCACGTAAAAAGGGTATCGATAAGCTTATGGCTGATGTTTGTATGGATCCTGAGCCCATAGTGCGTATC...
            (RBS)      Hind III
```

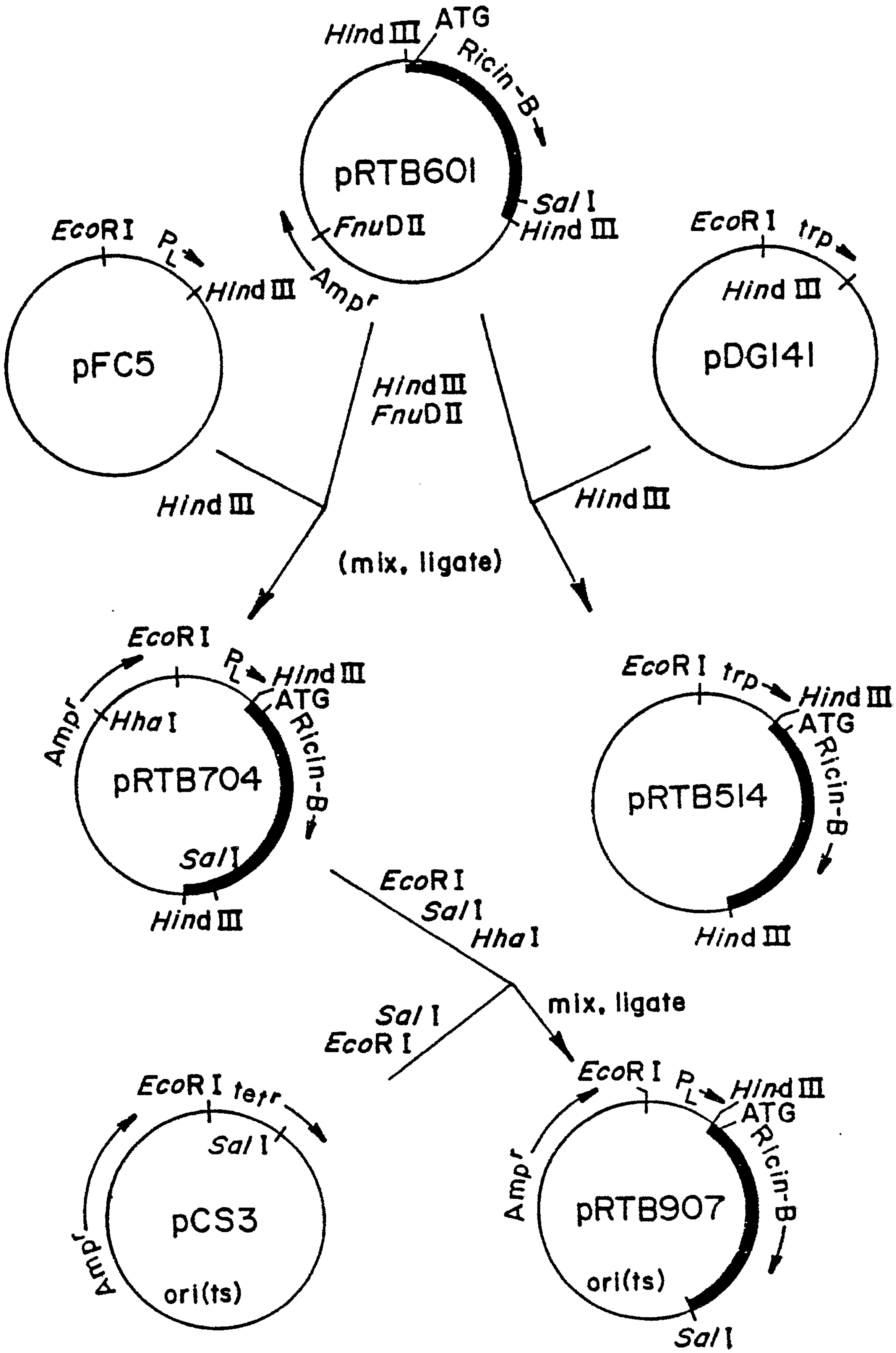

HindIII
ATG
Ricin-B
pRTB601
SalI
HindIII
FnuDII
Amp^r
EcoRI
P_L
HindIII
pFC5
EcoRI
trp
HindIII
pDG141
HindIII
FnuDII
HindIII
HindIII
(mix, ligate)
EcoRI
P_L
HindIII
ATG
Ricin-B
Amp^r
HhaI
pRTB704
SalI
HindIII
EcoRI
trp
HindIII
ATG
Ricin-B
pRTB514
HindIII
EcoRI
SalI
HhaI
SalI
EcoRI
mix, ligate
EcoRI
tetr
SalI
Amp^r
pCS3
ori(ts)
EcoRI
P_L
HindIII
ATG
Ricin-B
Amp^r
pRTB907
ori(ts)
SalI


FIG. 13

```
                                                      (HindIII)
                                    (1) ccaagaattgctgcaaaagcttatgaaaccggg
   TCTTCCTCAGCTGCTCACTTTCCAATAAAATTCCAAGAATTGCTGCAATCAAAGATGAAACCGGGAGGAAATACT
                                                         METLysProGlyGlyAsnThr

                                               BamH1        (2) ctttcacattagag
   ATTGTAATATGGATGTATGCAGTGGCAACATGGCTTTGTTTTGGATCCACCTCAGGGTGGTCTTTCACATTAGAG
   IleValIleTrpMETTyrAlaValAlaThrTrpLeuCysPheGlySerThrSerGlyTrpSerPheThrLeuGlu
  (HindIIIMET)                                          --- ←-----(leader) ←-----
   aagcttatgatattccccaaac
   GATAACAACATATTCCCCAAACAATACCCAATTATAAACTTTACCACAGCGGGTGCCACTGTGCAAAGCTACACA
   AspAsnAsnIlePheProLysGlnTyrProIleIleAsnPheThrThrAlaGlyAlaThrValGlnSerTyrThr
RTA- ←-------IlePheProLysGlnTyrProIleIleAsnPheThrThrAlaGlyAlaThrValGlnSerTyrThr

   AACTTTATCAGAGCTGTTCGCGGTCGTTTAACAACTGGAGCTGATGTGAGACATGAAATACCAGTGTTGCCAAAC
   AsnPheIleArgAlaValArgGlyArgLeuThrThrGlyAlaAspValArgHisGluIleProValLeuProAsn
RTA-AsnPheIleArgAlaValArgGlyArgLeuThrThrGlyAlaAspValArgHisGluIleProValLeuProAsn

   AGAGTTGGTTTGCCTATAAACCAACGGTTTATTTTAGTTGAACTCTCAAATCATGCAGAGCTTTCTGTTACATTA
   ArgValGlyLeuProIleAsnGlnArgPheIleLeuValGluLeuSerAsnHisAlaGluLeuSerValThrLeu
RTA-ArgValGlyLeuProIleAsnGlnArgPheIleLeuValGluLeuGlnAsnHisAlaGluIleSerValThrLeu

   GCGCTGGATGTCACCAATGCATATGTGGTAGGCTACCGTGCTGGAAATAGCGCATATTTCTTTCATCCTGACAAT
   AlaLeuAspValThrAsnAlaTyrValValGlyTyrArgAlaGlyAsnSerAlaTyrPhePheHisProAspAsn
RTA-AlaLeuSerValThrAsnAlaTyrValValGlyTyrArgAlaGlyAsnSerAlaTyrPhePheHisProAspAsn

   CAGGAAGATGCAGAAGCAATCACTCATCTTTTCACTGATGTTCAAAATCGATATACATTCGCCTTTGGTGGTAAT
   GlnGluAspAlaGluAlaIleThrHisLeuPheThrAspValGlnAsnArgTyrThrPheAlaPheGlyGlyAsn
RTA-GlnGluAspAlaGluAlaIleThrHisLeuPheThrAspValGlnAsnArgTyrThrPheAlaPheGlyGlyAsn

   TATGATAGACTTGAACAACTTGCTGGTAATCTGAGAGAAAATATCGAGTTGGGAAATGGTCCACTAGAGGAGGCT
   TyrAspArgLeuGluGlnLeuAlaGlyAsnLeuArgGluAsnIleGluLeuGlyAsnGlyProLeuGluGluAla
RTA-TyrAspArgLeuGluGlnLeuAlaGlyAsnLeuArgGluAsnIleGluLeuGlyAsnGlyProLeuGluGluAla

   ATCTCAGCGCTTTATTATTACAGTACTGGTGGCACTCAGCTTCCAACTCTGGCTCGTTCCTTTATAATTTGCATC
   IleSerAlaLeuTyrTyrTyrSerThrGlyGlyThrGlnLeuProThrLeuAlaArgSerPheIleIleCysIle
RTA-IleSerAlaLeuTyrTyrTyrSerThrGlyGlyThrGlnLeuProThrLeuAlaArgSerPheIleIleCysIle

   CAAATGATTTCAGAAGCAGCAAGATTCCAATATATTGAGGGAGAAATGCGCACGAGAATTAGGTACAACCGGAGA
   GlnMETIleSerGluAlaAlaArgPheGlnTyrIleGluGlyGluMETArgThrArgIleArgTyrAsnArgArg
RTA-GlnMetIleSerGluAlaAlaArgPheGlnTyrIleGluGlyGluMetArgThrArgIleArgTyrAsnArgArg

   TCTGCACCAGATCCTAGCGTAATTACACTTGAGAATAGTTGGGGGAGACTTTCCACTGCAATTCAAGAGTCTAAC
   SerAlaProAspProSerValIleThrLeuGluAsnSerTrpGlyArgLeuSerThrAlaIleGlnGluSerAsn
RTA-SerAlaProAspProSerValIleThrLeuGluAsnSerTrpGlyArgLeuSerThrAlaIleGlnGluSerAsn

   CAAGGAGCCTTTGCTAGTCCAATTCAACTGCAAAGACGTAATGGTTCCAAATTCAGTGTGTACGATGTGAGTATA
   GlnGlyAlaPheAlaSerProIleGlnLeuGlnArgArgAsnGlySerLysPheSerValTyrAspValSerIle
RTA-GlnGlyAlaPheAlaSerProIleGlnLeuGlnArg   AspGlySerLysPheSerValTyrAspValSerIle
                                                                    (TER)
                                                    (3) cacagttttaattgcttataagg
   TTAATCCCTATCATAGCTCTCATGGTGTATAGATGCGCACCTCCACCATCGTCACAGTTTTCTTTGCTTATAAGG
   LeuIleProIleIleAlaLeuMETValTyrArgCysAlaProProProSerSerGlnPheSerLeuLeuIleArg
RTA-LeuLeuProIleIleAla   MetValTyrArgCysAlaProProProSerSerGlnPhe(<--A-chain)

                                  BamH1
   CCAGTGGTACCAAATTTTAATGCTGATGTTTGTATGGATCCTGAGCCCATAGTGCGTATCGTAGGTCGAAATGGT
   ProValValProAsnPheAsnAlaAspValCysMETAspProGluProIleValArgIleValGlyArgAsnGly
RTB-         (B-chain-> )AlaAspValCysMetAspProGluProIleValArgIleValGlyArgAsnGly
```

FIG. 14

## Estimated Ricin Agglutinin A Sequence

```
RTA-IlePheProLysGlnTyrProIleIleAsnPheThrThrAlaGlyAlaThrValGlnSerTyrThrAsnPheIle

        CTGTGCGCAGTCATTTAACAACTGGAGGTGATGTGAGACATGAAATACCAGTGTTGCCAAACAGAGTTGGT
          ValArgSerHisLeuThrThrGlyGlyAspValArgHisGluIleProValLeuProAsnArgValGly
RTA-ArgAlaValArgGlyArgLeuThrThrGlyAlaAspValArgHisGluIleProValLeuProAsnArgValGly

    TTGCCTATAAGCCAACGGTTTATTTTAGTTGAACTCTCAAATCATGCAGAGCTTTCTGTTACATTAGCACTGGAT
    LeuProIleSerGlnArgPheIleLeuValGluLeuSerAsnHisAlaGluLeuSerValThrLeuAlaLeuAsp
RTA-LeuProIleAsnGlnArgPheIleLeuValGluLeuGlnAsnHisAlaGluIleSerValThrLeuAlaLeuSer

    GTCACCAATGCATATGTGGTCGGCTGCCGCGCTGGAAATAGCGCCTATTTCTTTCATCCTGACAATCAAGAAGAT
    ValThrAsnAlaTyrValValGlyCysArgAlaGlyAsnSerAlaTyrPhePheHisProAspAsnGlnGluAsp
RTA-ValThrAsnAlaTyrValValGlyTyrArgAlaGlyAsnSerAlaTyrPhePheHisProAspAsnGlnGluAsp

    GCAGAAGCAATCACTCATCTTTTCACGGATGTTCAAATT??????????????GCTTTTGGTGGTAATTATGATAGA
    AlaGluAlaIleThrHisLeuPheThrAspValGlnIle?????????????AlaPheGlyGlyAsnTyrAspArg
RTA-AlaGluAlaIleThrHisLeuPheThrAspValGlnAsnArgTyrThrPheAlaPheGlyGlyAsnTyrAspArg

    CTTGAACAACTTG?AGGT   CTTGAGAGAAATATTGAGTTGGGAACTGGTCCATTAGAGGACGCTATCTCAGCG
    LeuGluGlnLeu???Gly   LeuGluArgAsnIleGluLeuGlyThrGlyProLeuGluAspAlaIleSerAla
RTA-LeuGluGlnLeuAlaGlyAsnLeuArgGluAsnIleGluLeuGlyAsnGlyProLeuGluGluAlaIleSerAla

    CTTTATTATTATAGTACTTGTGGCACTCAGATTCCAACTCTGGCTCGTTCCTTTATGGTTTGCATCCAAATGATT
    LeuTyrTyrTyrSerThrCysGlyThrGlnIleProThrLeuAlaArgSerPheMETValCysIleGlnMETIle
RTA-LeuTyrTyrTyrSerThrGlyGlyThrGlnLeuProThrLeuAlaArgSerPheIleIleCysIleGlnMetIle

    TCAGAAGCAGCAAGATTCCAGTACATTGAGGGAGAAATGCGCACGAGAATTAGGTACAACCGAAGATCTGCACCA
    SerGluAlaAlaArgPheGlnTyrIleGluGlyGluMETArgThrArgIleArgTyrAsnArgArgSerAlaPro
RTA-SerGluAlaAlaArgPheGlnTyrIleGluGlyGluMetArgThrArgIleArgTyrAsnArgArgSerAlaPro

    GATCCTAGCGTAATTACACTTGAGAATAGTTGGGGGAGACTTTCCACTGCAATTCAAGAGTCTAACCAAGGAGCC
    AspProSerValIleThrLeuGluAsnSerTrpGlyArgLeuSerThrAlaIleGlnGluSerAsnGlnGlyAla
RTA-AspProSerValIleThrLeuGluAsnSerTrpGlyArgLeuSerThrAlaIleGlnGluSerAsnGlnGlyAla

    TTTGCTAGTCCAATTCAACTGCAAAGACGTAACGGTTCCAAATTCAATGTGTACGATGTGAGTATATTAATCCCT
    PheAlaSerProIleGlnLeuGlnArgArgAsnGlySerLysPheAsnValTyrAspValSerIleLeuIlePro
RTA-PheAlaSerProIleGlnLeuGlnArg   AspGlySerLysPheSerValTyrAspValSerIleLeuLeuPro

    ATCATAGCTCTCATGGTGTATAGATGCGCACCTCCACCGTCGTCACAGTTTTCTTTGCTTATAAGGCCAGTGGTG
    IleIleAlaLeuMETValTyrArgCysAlaProProProSerSerGlnPheSerLeuLeuIleArgProValVal
RTA-IleIleAla   MetValTyrArgCysAlaProProProSerSerGlnPhe
                                           (A chain <-- )

    CCAAATTTTAATGCTGATGTTTGTATGGATCCTGAGCCCATAGTGCGTATCGTAGGTCGAAATGGTCTATGTGTT
    ProAsnPheAsnAlaAspValCysMETAspProGluProIleValArgIleValGlyArgAsnGlyLeuCysVal
RTA-            AlaAspValCysMetAspProGluProIleValArgIleValGlyArgAsnGlyLeuCysVal
               ( -> B chain)

    GATGTTACAGGTGAAGAATTC
    AspValThrGlyGluGluPhe
RTB-AsnValArgAspGlyArgPhe
```

This is a composite derived from the inserts of pRTA115 and pRA45.
? = undetermined sequence

FIG. 15

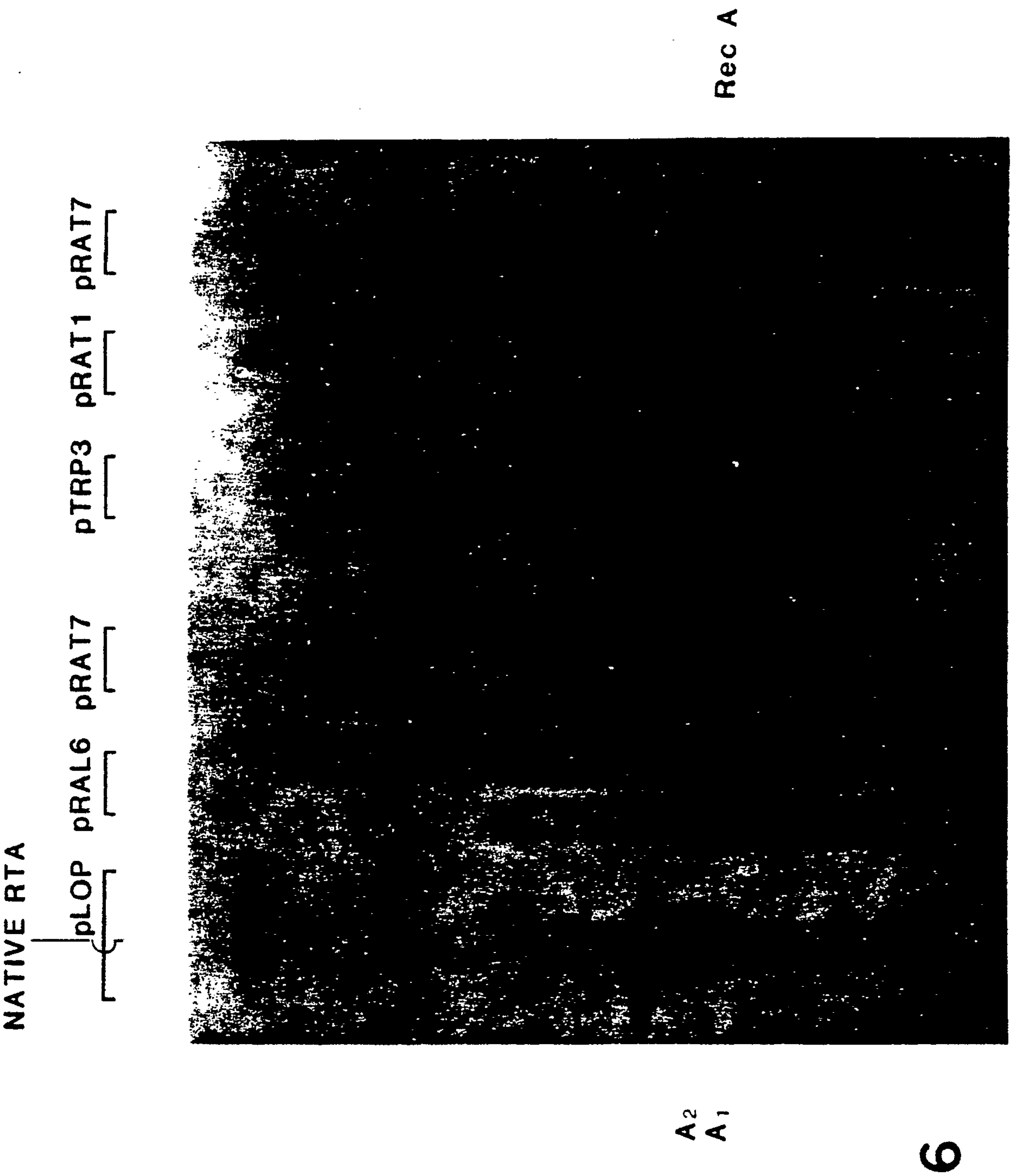


FIG. 16

# FIG. 17

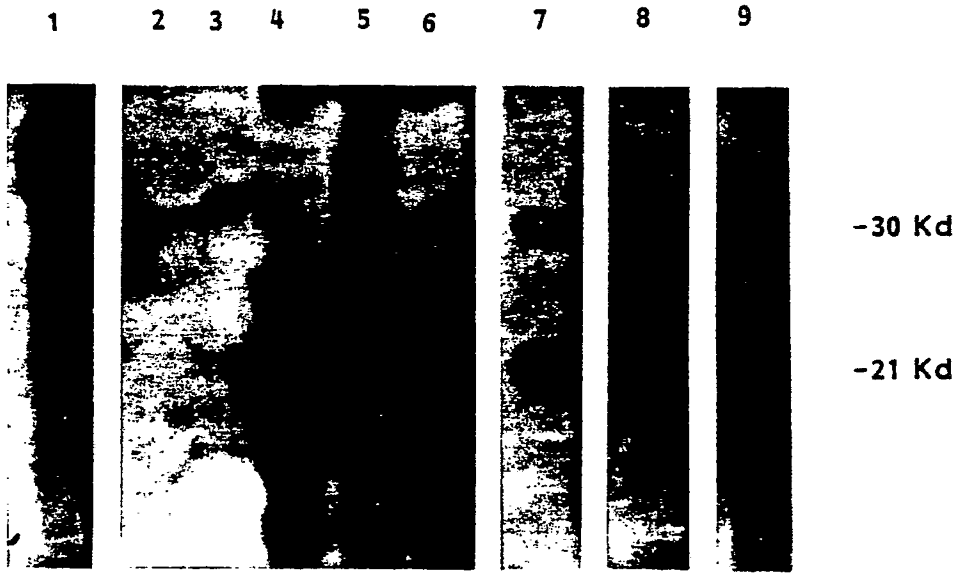


Key

1 natural ricin-B

2 pUC8

3 pRTB5

4 pRTB151

5 pRTB221

6 pRTB236

7 pRTB514

8 pRTB704

9 pRTB907